# EUROPÄISCHE PATENTSCHRIFT

(11) **EP 1 202 969 B1**
(45) Veröffentlichungstag und Bekanntmachung des Hinweises auf die Patenterteilung: **09.03.2005**
(21) Anmeldenummer: 00951389.6
(22) Anmeldetag: 15.07.2000
(51) Int. Cl.: C07D 211/58, A61K 31/4468, A61P 9/10

(54) **BIPHENYLDERIVATE, IHRE HERSTELLUNG UND IHRE VERWENDUNG ALS MTP-INHIBITOR**
BIPHENYL DERIVATIVES, PRODUCTION THEREOF AND USE AS MEDICINES
DERIVES DE BIPHENYLE, LEUR PRODUCTION ET LEUR UTILISATION COMME MEDICAMENTS

(30) Priorität: 20.07.1999 DE 19933926
(43) Veröffentlichungstag der Anmeldung: 08.05.2002
(73) Patentinhaber: Boehringer Ingelheim Pharma GmbH & Co.KG, 55218 Ingelheim am Rhein (DE)
(72) Erfinder: LEHMANN-LINTZ, Thorsten, D-88416 Ochsenhausen/Laubach (DE); HECKEL, Armin, D-88400 Biberach (DE); THOMAS, Leo, D-88400 Biberach (DE); MARK, Michael, D-88400 Biberach (DE)
(86) Internationale Anmeldenummer: PCT/EP2000/006770
(87) Internationale Veröffentlichungsnummer: WO 2001/005762

(56) Entgegenhaltungen:
- EP-A- 0 887 345
- WO-A-99/63929

## Beschreibung

Gegenstand der vorliegenden Erfindung sind Biphenylderivate der allgemeinen Formel 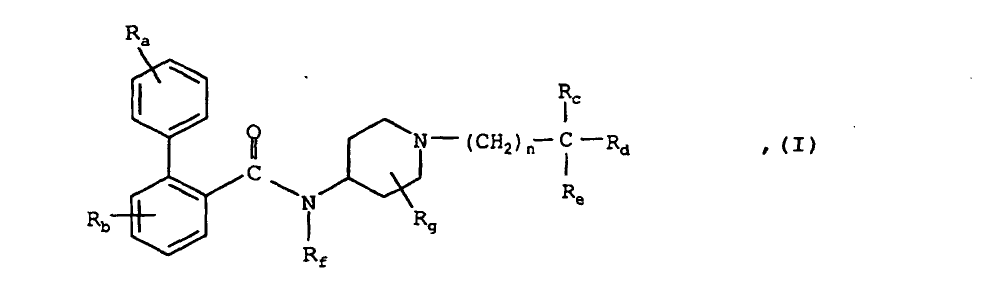 deren Isomere, deren Salze, insbesondere deren physiologisch verträgliche Salze, welche wertvolle pharmakologische Eigenschaften aufweisen.

Die Verbindungen der obigen allgemeinen Formel I stellen wertvolle Inhibitoren des mikrosomalen Triglyzerid-Transferproteins (MTP) dar und eignen sich daher zur Senkung der Plasmaspiegel der atherogenen Lipoproteine. Proteine mit ähnlicher Wirkung werden z.B. in WO 99/63929 und EP-887345 beschrieben

In der obigen allgemeinen Formel I bedeutet
n die Zahl 1, 2, 3, 4 oder 5,
Rₐ und R_{b}, die gleich oder verschieden sein können, jeweils ein Wasserstoff-, Fluor-, Chlor- oder Bromatom, eine C₁₋₃-Alkylgruppe, in der die Wasserstoffatome ganz oder eine C₁₋₁₀-Alkyl-, C₃₋₇-Cycloalkyl- oder C₃₋₇-Cycloalkyl-C₁₋₃-alkylgruppe, in denen jeweils die Wasserstoffatome ganz oder teilweise durch Fluoratome ersetzt sein können,
eine gegebenenfalls durch ein Fluor-, Chlor- oder Bromatom, durch eine C₁₋₃-Alkylgruppe, in der die Wasserstoffatome ganz oder teilweise durch Fluoratome ersetzt sein können, durch eine Hydroxy-, C₁₋₃-Alkoxy-, Carboxy-, C₁₋₃-Alkoxycarbonyl-, Aminocarbonyl-, C₁₋₃-Alkylaminocarbonyl- oder N,N-Di-(C₁₋₃-Alkyl)-aminocarbonylgruppe, durch eine 3- bis 7-gliedrige Cycloalkyleniminogruppe, wobei die Methylengruppe in Position 4 in einer 6- oder 7-gliedrigen Cycloalkyleniminogruppe zusätzlich durch ein Sauerstoff- oder Schwefelatom, durch eine Sulfinyl-, Sulfonyl-, Imino- oder N-(C₁₋₃-Alkyl)-iminogruppe ersetzt sein kann, durch eine Nitro-, Amino-, C₁₋₃-Alkylamino-, Di- (C₁₋₃-Alkyl)-amino-, C₁₋₃-Alkylcarbonylamino-, N- (C₁₋₃-Alkyl) - C₁₋₃-alkylcarbonylamino-, C₁₋₃-Alkylsulfonylamino- oder N-(C₁₋₃-Alkyl)-C₁₋₃-alkylsulfonylaminogruppe substituierte Phenyl-, Naphthyloder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und .ein Sauerstoff- oder Schwefelatom oder ein oder zwei Stickstoffatome enthält, und außerdem an die vorstehend erwähnten monocyclischen heterocyclischen Gruppen über zwei benachbarte Kohlenstoffatome ein Phenylring ankondensiert sein kann,
R_{d} eine gegebenenfalls durch ein Fluor-, Chlor- oder Bromatom, durch eine C₁₋₃-Alkylgruppe, in der die Wasserstoffatome ganz oder teilweise durch Fluoratome ersetzt sein können, durch eine Hydroxy-, C₁₋₃-Alkoxy-, Carboxy-, C₁₋₃-Alkoxycarbonyl-, Aminocarbonyl-, C₁₋₃-Alkylaminocarbonyl- oder N,N-Di- (C₁₋₃-Alkyl)-aminocarbonylgruppe, durch eine 3- bis 7-gliedrige Cycloalkyleniminogruppe, wobei die Methylengruppe in Position 4 in einer 6- oder 7-gliedrigen Cycloalkyleniminogruppe zusätzlich durch ein Sauerstoff- oder Schwefelatom, durch eine Sulfinyl-, Sulfonyl-, Imino- oder N-(C₁₋₃-Alkyl)-iminogruppe ersetzt sein kann, durch eine Nitro-, Amino-, C₁₋₃-Alkylamino-, Di-(C₁₋₃-Alkyl)-amino-, C₁₋₃-Alkylcarbonylamino- , N-(C₁₋₃-Alkyl)-C₁₋₃-alkylcarbonylamino-, C₁₋₃-Alkylsulfonylamino- oder N-(C₁₋₃-Alkyl)-C₁₋₃-alkylsulfonylaminogruppe substituierte Phenyl-, Naphthyloder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält, und außerdem an die vorstehend erwähnten monocyclischen heterocyclischen Gruppen über zwei benachbarte Kohlenstoffatome ein Phenylring ankondensiert sein kann,
Rₑ eine Carboxygruppe, eine C₁₋₆-Alkoxycarbonyl- oder C₃₋₇-Cycloalkoxycarbonylgruppe, in denen der Alkyl- oder Cycloalkylteil jeweils ab Position 2 bezogen auf das Sauerstoffatom durch eine C₁₋₃-Alkoxy-, Amino-, C₁₋₃-Alkylamino- oder Di- (C₁₋₃-alkyl)-aminogruppe substituiert sein kann, eine Phenyl-C₁₋₃-alkoxycarbonyl- oder Heteroaryl-C₁₋₃-alkoxycarbonylgruppe, wobei der Heteroarylteil wie vorstehend erwähnt definiert ist,
R_{f} ein Wasserstoffatom, eine C₁₋₃-Alkyl- oder Phenyl-C₁₋₃-alkylgruppe und
R_{g} ein Wasserstoffatom oder eine C₁₋₃-Alkylgruppe.

Bevorzugte Verbindungen der obigen allgemeinen Formel I sind jedoch diejenigen, in denen
R_{b} bis R_{g} wie eingangs erwähnt definiert sind und
Rₐ in 3' oder 4'-Stellung steht und mit Ausnahme des Wasser stoffatoms wie eingangs erwähnt definiert ist,
deren isomere und deren Salze.

Besonders bevorzugte Verbindungen der obigen allgemeinen Formel I sind jedoch diejeigen, in denen
n die Zahl 3 , 4 oder 5,
Rₐ ein Wasserstoff-, Fluor-, Chlor- oder Bromatom, eine C₁₋₃-Alkyl-, Trifluormethyl- oder C₁₋₃-Alkoxygruppe,
R_{b} ein Wasserstoff-, Fluor-, Chlor- oder Bromatom,
R_{c} eine C₁₋₅-Alkyl-, C₃₋₇-Cycloalkyl- oder C₃₋₇-Cycloalkyl-C₁₋₃-alkylgruppe oder
eine gegebenenfalls durch ein Fluor-, Chlor- oder Bromatom oder durch eine C₁₋₃-Alkylgruppe substituierte Phenylgruppe,
R_{d} eine gegebenenfalls durch ein Fluor-, Chlor- oder Bromatom oder durch eine C₁₋₃-Alkylgruppe substituierte Phenylgruppe,
Rₑ eine Carboxygruppe, eine C₁₋₆-Alkoxycarbonyl- oder C₃₋₇-Cycloalkoxycarbonylgruppe, in denen der Alkyl- oder Cycloalkylteil jeweils ab Position 2 bezogen auf das Sauerstoffatom durch eine C₁₋₃-Alkoxy-, Amino-, C₁₋₃-Alkylamino- oder Di-(C₁₋₃-alkyl)-aminogruppe substituiert sein kann, eine Phenyl-C₁₋₃-alkoxycarbonyl-, Pyridyl-C₁₋₃-alkoxycarbonyl- oder Pyrimidyl-C₁₋₃-alkoxycarbonylgruppe,
R_{f} ein Wasserstoffatom oder eine C₁₋₃-Alkylgruppe und
R_{g} ein Wasserstoffatom oder eine C₁₋₃-Alkylgruppe bedeuten,
deren Isomere und deren Salze.

Als besonders wervolle Verbindungen seien beispielsweise folgende erwähnt:
(a) 2-Methyl-2-phenyl-5-{4-[(4'-trifluormethyl-biphenyl-2-carbonyl)-amino]-piperidin-1-yl}-pentancarbonsäure-methylester,
(b) 2-Ethyl-2-phenyl-5-{4-[(4'-trifluormethyl-biphenyl-2-carbonyl)-amino]-piperidin-1-yl}-pentancarbonsäure-methylester und
(c) 2-Methyl-2-phenyl-6-{4-[(4'-trifluormethyl-biphenyl-2-carbonyl)-amino]-piperidin-1-yl}-hexancarbonsäuremethylester.

Erfindungsgemäß erhält man die neuen Verbindungen nach literaturbekannten Verfahren, beispielsweise nach folgenden Verfahren:
a. Umsetzung einer Verbindung der allgemeinen Formel 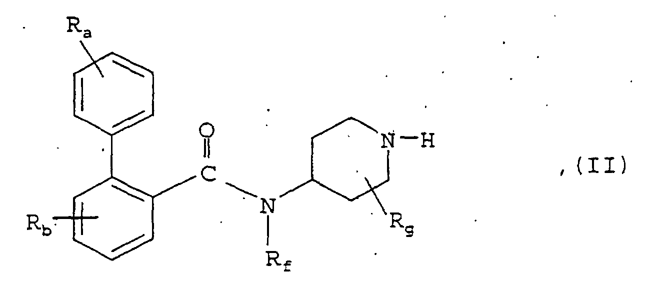 in der
   Rₐ, R_{b}, R_{f} und R_{g} wie eingangs erwähnt definiert sind, mit einer Verbindung der allgemeinen Formel 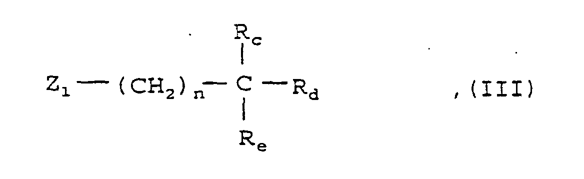 in der
   n und R_{c} bis Rₑ wie eingangs erwähnt definiert sind und
   Z₁ eine nukleofuge Austrittsgruppe wie ein Halogenatom, z.B. ein Chlor-, Brom- oder Jodatom, oder eine p-Nitrophenylgruppe bedeutet.
   Die Umsetzung wird vorzugsweise in einem Lösungsmittel wie Methylenchlorid, Acetonitril, Tetrahydrofuran, Toluol, Aceton/-Wasser, Dimethylformamid oder Dimethylsulfoxid gegebenenfalls in Gegenwart einer Base wie Natrimhydrid, Kaliumcarbonat, Kalium-tert.butylat oder N-Ethyl-diisopropylamin bei Temperaturen zwischen 0 und 100°C, vorzugsweise bei Temperaturen zwischen 10 und 60°C, durchgeführt.
b. Zur Herstellung einer Verbindung der allgemeinen Formel I, in der Rₑ mit Ausnahme der Carboxygruppe die für Rₑ eingangs erwähnten Bedeutungen aufweist:
   Veresterung einer Verbindung der allgemeinen Formel 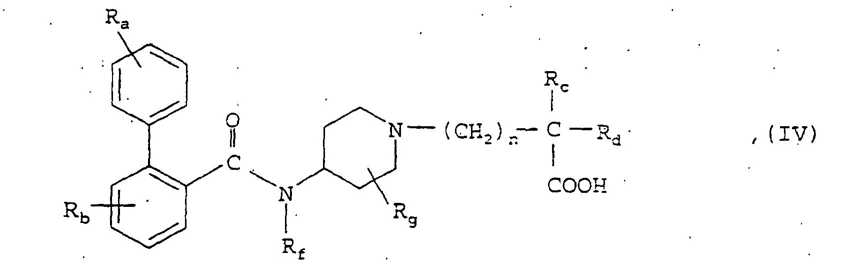 in der
   n, Rₐ bis R_{d}, R_{f} und R_{g} wie eingangs erwähnt definiert sind, oder deren reaktionsfähigen Derivate mit einem Alkohol der allgemeinen Formel

   H - Rₑ' , (V)

   in der
   Rₑ' eine C₁₋₆-Alkoxy- oder C₃₋₇-Cycloalkoxygruppe, in denen der Alkyl- oder Cycloalkylteil jeweils ab Position 2 bezogen auf das Sauerstoffatom durch eine C₁₋₃-Alkoxy-, Amino-, C₁₋₃-Alkylamino- oder Di-(C₁₋₃-alkyl -aminogruppe substituiert sein kann, eine Phenyl-C₁₋₃-alkoxy- oder Heteroaryl-C₁₋₃-alkoxygruppe, wobei der Heteroarylteil wie vorstehend erwähnt definiert ist, bedeutet, oder zur Herstellung eines tert.Butylesters auch 2,2-Dimethyl-ethen in Gegegenwart einer Säure.
   Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, vorzugsweise jedoch in einem Überschuß des eingesetzten Alkohols der allgemeinen Formel V als Lösungsmittel, gegebenenfalls in Gegenwart einer Säure wie Salzsäure oder Schwefelsäure oder in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2- (1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.
   Die Umsetzung einer entsprechenden reaktionsfähigen Verbindung der allgemeinen Formel IV wie deren Ester, Imidazolide oder Halogeniden mit einem Alkohol der allgemeinen Formel V wird vorzugsweise in einem entsprechenden Alkohol als Lösungsmittel gegebenenfalls in Gegenwart eines weiteren Lösungsmittels wie Methylenchlorid oder Ether und vorzugsweise in Gegenwart einer tertiären organische Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.
   Die tert.Butylesterbildung mit 2,2-Dimethyl-ethen wird vorzugsweise in einem Lösungsmittel wie Diethylether, Dioxan, Methylenchlorid oder tert.Butanol in Gegenwart einer Säure wie Schwefelsäure, Salzsäure oder Borfluorid-diethyletherat bei Temperaturen zwischen -20 und 150°C, vorzugsweise bei Temperaturen zwischen 0 und 100°C, durchgeführt.
c. Zur Herstellung einer Verbindung der allgemeinen Formel I, in der Rₑ eine Carboxygruppe darstellt:
   Überführung einer Verbindung der allgemeinen Formel 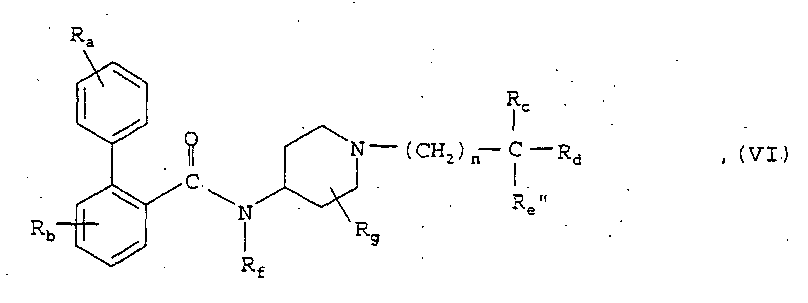 in der
   n, Rₐ bis R_{d}, R_{f} und R_{g} wie eingangs erwähnt definiert sind und Rₑ" eine in eine Carboxygruppe überführbare Gruppe darstellt, in eine Verbindung der allgemeinen Formel I, in der Rₑ eine Carboxygruppe darstellt.
   Als eine in eine Carboxygruppe überführbare Gruppe kommt beispielsweise eine durch einen Schutzrest geschützte Carboxylgruppe wie deren funktionelle Derivate, z. B. deren unsubstituierte oder substituierte Amide, Ester, Thioester, Trimethylsilylester, Orthoester oder Iminoester, welche zweckmäßigerweise mittels Hydrolyse in eine Carboxylgruppe übergeführt werden,
   deren Ester mit tertiären Alkoholen, z.B. der tert. Butylester, welche zweckmäßigerweise mittels Behandlung mit einer Säure oder Thermolyse in eine Carboxylgruppe übergeführt werden, und
   deren Ester mit Aralkanolen, z.B. der Benzylester, welche zweckmäßigerweise mittels Hydrogenolyse in eine Carboxylgruppe übergeführt werden, in Betracht.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet Rₑ" in einer Verbindung der Formel VI beispielsweise die tert. Butyloxycarbonylgruppe, so kann diese auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.

Bedeutet Rₑ" in einer Verbindung der Formel VI beispielsweise die Benzyloxycarbonylgruppe, so kann diese auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid 'vorzugsweise bei Temperaturen zwischen 0 und 50°C, z.B. bei Raumtemperatur, und einem Wasserstoffdruck von 1 bis 5 bar abgespalten werden.

Erhält man erfindungsgemäß eine Verbindung der allgemeinen Formel I, die eine Nitrogruppe enthält, so kann diese mittels Reduktion in eine entsprechende Aminoverbindung übergeführt werden oder
eine Verbindung der allgemeinen Formel I, in der R_{f} ein Wasserstoffatom darstellt, so kann diese mittels Alkylierung in eine entsprechende Verbindung, in der R_{f} eine C₁₋₃-Alkyl- oder Phenyl-C₁₋₃-alkylgruppe darstellt, übergeführt werden.

Die anschließende Reduktion einer Nitrogruppe wird zweckmäßigerweise hydrogenolytisch, z.B. mit Wasserstoff in Gegenwart eines Katalysators wie Platin, Palladium/Kohle oder Raney-Nickel in einem geeigneten Lösungsmittel wie Methanol, Ethanol, Essigsäureethylester, Tetrahydrofuran, Dioxan; Dimethylformamid oder Eisessig gegebenenfalls unter Zusatz einer Säure wie Salzsäure und bei einem Wasserstoffdruck von 1 bis 7 bar, vorzugsweise jedoch von 1 bis 5 bar, mit Metallen wie Eisen, Zinn oder Zink in Gegenwart einer Säure wie Essigsäure oder Salzsäure, mit Salzen wie Eisen (II) sulfat, Zinn (II) chlorid, Natriumsulfid, Natriumhydrogensulfit oder Natriumdithionit, oder mit Hydrazin in Gegenwart von Raney-Nickel bei Temperaturen zwischen 0 und 100°C, vorzugsweise jedoch bei Temperaturen zwischen 20 und 60°C, durchgeführt.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran, Dioxan, Dimethylsulfoxid oder Sulfolan mit einem Alkylierungsmittel wie einem entsprechenden Halogenid oder Sulfonsäureester, z.B. mit Methyljodid, Ethylbromid, Dimethylsulfat oder Benzylchlorid, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, oder mittels reduktiver Alkylierung durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, tert.Butyl-dimethylsilyl-, Acetyl-, Benzoyl-, Methyl-, Ethyl-, tert.Butyl-, Trityl-, Benzyl- oder Tetrahydropyranylgruppe,
als Schutzreste für eine Carboxygruppe die Trimethylsilyl-, Methyl-, Ethyl-, tert.Butyl-, Benzyl- oder Tetrahydropyranylgruppe und
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.
Die Abspaltung einer Silylgruppe kann jedoch auch mittels Tetrabutylammoniumfluorid wie vorstehend beschrieben erfolgen.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl-oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an. chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+) - oder (-) -Menthol und als optisch aktiver Acylrest in Amiden beispielsweise (+) -oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine saure Gruppe wie eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II bis VI sind literaturbekannt oder man erhält diese nach literaturbekannten Verfahren bzw. werden in den Beispielen beschrieben. So erhält man beispielsweise eine Verbindung der allgemeinen Formel III durch Veresterung einer entsprechenden disubstituierten Carbonsäure und anschlißende Umsetzung mit einem α,ω-Dihaogelalkan in Gegenwart einer starken Base wie Lithiumdiisopropylamid, Natriumamid oder Natriumhydrid.

Wie bereits eingangs erwähnt, weisen die Verbindungen der allgemeinen Formel I und deren physiologisch verträgliche Salze wertvolle pharmakologische Eigenschaften auf. Diese stellen insbesondere wertvolle Inhibitoren des mikrosomalen Triglyzerid-Transferproteins (MTP) dar und eignen sich daher zur Senkung der Plasmaspiegel der atherogenen Lipoproteine.

Beispielsweise wurden die erfindungsgemäßen Verbindungen auf ihre biologischen Wirkungen wie folgt untersucht:

Inhibitoren von MTP wurden durch einen kommerziell erhältlichen MTP-Aktivitäts-Kit identifiziert (WAK-Chemie Medical GmbH, Sulzbacherstrasse 15-21, D-65812 Bad Soden, Germany). Dieser Testkit enthält Donor- und Akzeptorpartikel. Die Donorpartikel enthalten Fluoreszenz-markierte Triglyzeride in einer Konzentration, die so hoch ist, daß eine Eigenlöschung der Fluoreszenz erfolgt. Bei Inkubation der Donor- und Akzeptorpartikel mit einer MTP-Quelle wurden Fluoreszenz-markierte Triglyzeride von den Donor- zu den Akzeptorpartikeln übertragen. Dies führte zu einem Anstieg der Fluoreszenz in der Probe. Solubilisierte Lebermikrosomen aus verschiedenen Spezies (z.B. Ratte) konnten als MTP-Quelle benutzt werden. Inhibitoren von MTP wurden als diejenigen Substanzen identifiziert, welche den Transfer von Fluoreszenz-markierten Triglyzeriden im Vergleich zu einem Kontrollansatz ohne Inhibitor erniedrigten.

Auf Grund der vorstehend erwähnten biologischen Eigenschaften eignen sich die Verbindungen der allgemeinen Formel I und deren physiologisch verträgliche Salze insbesondere zur Senkung der Plasmakonzentration von atherogenen Apolipoprotein B (apoB) -haltigen Lipoproteinen wie Chylomikronen und/oder Lipoproteinen sehr niedriger Dichte (VLDL) sowie deren Überreste wie Lipoproteine niedriger Dichte (LDL) und/oder Lipoprotein(a) (Lp (a) ) , zur Behandlung von Hyperlipidämien, zur Vorbeugung und Behandlung der Atherosklerose und ihrer klinischen Folgen, und zur Vorbeugung und Behandlung verwandter Erkrankungen wie Diabetes mellitus, Adipositas und Pankreatitis, wobei die orale Applikation bevorzugt ist.

Die zur Erzielung einer entsprechenden Wirkung erforderliche Tagesdosis liegt beim Erwachsenen zwischen 0,5 und 500 mg, zweckmäßigerweise zwischen 1 und 350 mg, vorzugsweise jedoch zwischen 5 und 200 mg.

Hierzu lassen sich die erfindungsgemäß hergestellten Verbindungen der Formel I, gegebenenfalls in Kombination mit anderen Wirksubstanzen wie anderen Lipidsenker, beispielsweise mit HMG-CoA-Reduktase-Inhibitoren, Cholesterolbiosynthese-Inhibitoren wie Squalensynthase-Inhibitoren und Squalenzyklase-Inhibitoren, Gallensäure-bindende Harze, Fibrate, Cholesterol-Resorptions-Inhibitoren, Niacin, Probucol, CETP Inhibitoren und ACAT Inhibitoren zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure; Wasser, Wasser/Ethanol, Wasser/Glycerin, Wasser/-Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung nähers erläutern :

### Beispiel 1

### 2-Methyl-2-phenyl-5-{4-[(4'-trifluormethyl-biphenyl-2-carbonyl)-amino]-piperidin-1-yl}-pentancarbonsäuremethylester

### a. 2-Phenylpropionsäuremethylester

50 g (0.3 Mol) 2-Phenylpropionsäure werden in 375 ml methanolischer Salzsäure gelöst und 14 Stunden bei Raumtemperatur gerührt. Das Lösungsmittel wird entfernt und der Rückstand mit Essigsäureethylester und gesättigter Natriumhydrogencarbonatlösung extrahiert. Die organischen Phasen werden mit Wasser und gesättigter Kochsalzlösung extrahiert, über Magnesiumsulfat getrocknet und eingedampft.
Ausbeute: 51 g (94.8% der Theorie).

### b. 5-Brom-2-methyl-2-phenyl-pentancarbonsäuremethylester

Zu einer Lösung von 32.8 ml (0.234 Mol) Diisopropylamin in 200 ml wasserfreiem Tetrahydrofuran werden bei -30°C 15 g (0.234 Mol) n-Butyllithium als 2.5 molare Lösung in Hexan zugetropft und 10 Minuten bei -10°C gerührt. Bei -76°C werden 38.4 g .(0.234 Mol) 2-Phenylpropionsäuremethylester zugetropft und 30 Minuten bei dieser Temperatur gerührt. Anschließend werden 26.3 ml (0.257 Mol) 1,3-Dibrompropan zugesetzt, nach beendeter Zugabe das Kühlbad entfernt und 14 Stunden bei Raumtemperatur gerührt. Die Reaktionslösung wird auf 1.2 l Wasser gegossen und mit Diethylether extrahiert. Die organischen Phasen werden mit Wasser extrahiert, über Natriumsulfat getrocknet und das Lösungsmittel entfernt. Der Rückstand wird im Hochvakuum destilliert.
Ausbeute: 42.7 g (64 % der Theorie),
Siedepunkt: 113-118°C bei 0.2 mmbar

### c. 4'-Trifluormethyl-biphenyl-2-carbonsäurechlorid

Zu einer Suspension von 30 g (0.113 Mol) 4'-Trifluormethylbiphenyl-2-carbonsäure in 500 ml Dichlormethan und 0.2 ml Dimethylformamid werden bei 0°C 170 ml Oxalylchlorid zugetropft. Nach Beendigung der Reaktion wird das Reaktionsgemisch durch Destillation eingeengt und das 4'-Trifluormethyl-biphenyl-2-carbonsäurechlorid als Rohprodukt weiter umgesetzt.
Ausbeute: 32.16 g (100 % der Theorie).

### d. 4'-Trifluoromethyl-biphenyl-2-carbonsäure-(1-benzyl-piperidin-4-yl)-amid

Zu einer Lösung von 22.43 ml (0.11 Mol) 4-Amino-1-benzyl-piperidin und 42.97 ml (0.31 Mol) Triethylamin in 600 ml Dichlormethan werden 34.1 g (0.12 Mol) 4'-Trifluormethyl-biphenyl-2-carbonsäurechlorid, gelöst in 300 ml Dichlormethan, bei 15 bis 20°C zugetropft. Es wird 14 Stunden bei Raumtemperatur gerührt. Die Reaktionlösung wird mit 300 ml Dichlormethan verdünnt und mit einer Mischung aus 1N Natriumhydrogencarbonatlösung und 1N Natronlauge extrahiert. Die organische Phase wird über Natriumsulfat getrocknet und das Lösungsmittel entfernt. Der Rückstand wird aus 650 ml Ethanol umkristallisiert.
Ausbeute: 31.6 g (65.5 % der Theorie),
Schmelzpunkt: 193°C
C₂₆H₂₅F₃N₂O (M = 438,50)
Ber.: Molpeak (M+H)⁺ = 439
Ge.f.: Molpeak (M+H)⁺ = 439

### e. 4'-Trifluormethyl-biphenyl-2-carbonsäure-piperidin-4-ylamid

31.6 g (0.072 Mol) 4'-Trifluoromethyl-biphenyl-2-carbonsäue-(1-benzyl-piperidin-4-yl)-amid werden in 180 ml Ethanol und 180 ml Methanol gelöst, mit 84 ml (0.83 Mol) Cyclohexen und 6,53 g Palladiumhydroxid (20%ig auf Kohle) versetzt und vier Stunden zum Rückfluß erhitzt. Die heiße Reaktionsmischung wird über Kieselgur filtriert und das Reaktionsgemisch am Rotationsverdampfer eingeengt.
Ausbeute : 18.5 g (73.7 % der Theorie),
C₁₉H₁₉F₃N₂O (M = 348,37)
Ber.: Molpeak (M+H)⁺ = 349
Gef.: Molpeak (M+H)⁺ = 349

### f. 2-Methyl-2-phenyl-5-{4-[(4'-trifluormethyl-biphenyl-2-carbonyl)-amino]-piperidin-1-yl}-pentancarbonsäure-methylester

0.611 g (1.75 mMol) 4' -Trifluormethyl-biphenyl-2-carbonsäurepiperidin-4-yl-amid, 0.5 g (1.75 mMol) 5-Brom-2-methyl-2-phenyl-pentancarbonsäuremethylester und 0.8 g (5.7 mMol) Kaliumcarbonat werden in 50 ml Dimethylformamid gelöst und mit Wasser versetzt. Es wird 72 Stunden bei 90°C gerührt. Im Anschluß wird das Reaktionsgemisch auf Wasser gegossen, der Niederschlag abfiltriert und getrocknet. Nach Säulenchromatographie an Kieselgel (Eluens: Essigsäureethylester) verbleiben farblose Kristalle.
Ausbeute: 0,15 g (15,5 % der Theorie),
C₃₂H₃₅F₃N₂O₃ (M = 552,64)
Schmelzpunkt: 139-140 °C
Ber. : Molpeak (M+H)⁺ = 553
Gef.: Molpeak (M+H)⁺ = 553

Analog Beispiel 1 können folgende Verbindungen hergestellt werden:
(1) 2-Methyl-2-phenyl-5-{4-[(4'-trifluormethyl-biphenyl-2-carbonyl)-amino]-piperidin-1-yl}-pentancarbonsäure-ethylester
(2) 2-Methyl-2-phenyl-5-{4-[(4'-trifluormethyl-biphenyl-2-carbonyl)-amino]-piperidin-1-yl}-pentancarbonsäure-propylester
(3). 2-Methyl-2-phenyl-5-{4-[(4'-trifluormethyl-biphenyl-2-carbonyl)-amino]-piperidin-1-yl}-pentancarbonsäure-butylester
(4) 2-Methyl-2-phenyl-5-{4-[(4'-trifluormethyl-biphenyl-2-carbonyl)-amino]-piperidin-1-yl}-pentancarbonsäure-pentylester
(5) 2-Methyl-2-phenyl-5-{4-[(4'-trifluormethyl-biphenyl-2-carbonyl)-amino]-piperidin-1-yl}-pentancarbonsäure-hexylester
(6) 2-Methyl-2-phenyl-5-{4-[(4'-trifluormethyl-biphenyl-2-carbonyl)-amino]-piperidin-1-yl}-pentancarbonsäure-isopropylester
(7) 2-Methyl-2-phenyl-5-{4-[(4'-trifluormethyl-biphenyl-2-carbonyl)-amino]-piperidin-1-yl}-pentancarbonsäure-tert.butylester
(8) 2-Methyl-2-phenyl-5-{4-[(4'-trifluormethyl-biphenyl-2-carbonyl)-amino]-piperidin-1-yl}-pentancarbonsäure-isobutylester
(9) 2-Methyl-2-phenyl-5-{4-[(4'-trifluormethyl-biphenyl-2-carbonyl)-amino)-piperidin-1-yl}-pentancarbonsäure-sec.butylester
(10) 2-Methyl-2-phenyl-5-{4-[(4'-trifluormethyl-biphenyl-2-carbonyl)-amino]-piperidin-1-yl}-pentancarbonsäure-cyclopropylester
(11) 2-Methyl-2-phenyl-5-{4-[(4'-trifluormethyl-biphenyl-2-carbonyl)-amino]-piperidin-1-yl}-pentancarbonsäure-cyclobutylester
(12) 2-Methyl-2-phenyl-5-{4-[(4'-crifluormethyl-biphenyl-2-carbonyl)-amino]-piperidin-1-yl}-pentancarbonsäure-cyclopentylester
(13) 2-Methyl-2-phenyl-5-{4-[(4'-trifluormethyl-biphenyl-2-carbonyl)-amino]-piperidin-1-yl}-pentancarbonsäure-cyclohexylester
(14) 2-Methyl-2-phenyl-5-{4-[(4'-trifluormethyl-biphenyl-2-carbonyl)-amino]-piperidin-1-yl}-pentancarbonsäure-cycloheptylester
(15) 2-Methyl-2-phenyl-5-{4-[(4'-trifluormethyl-biphenyl-2-carbonyl)-amino]-piperidin-1-yl}-pentancarbonsäure-2-hydroxyethylester
(16) 2-Methyl-2-phenyl-5-{4-[(4'-trifluormethyl-biphenyl-2-carbonyl)-amino]-piperidin-1-yl}-pentancarbonsäure-3-hydroxypropylester
(17) 2-Methyl-2-phenyl-5-{4-[(4'-trifluormethyl-biphenyl-2-carbonyl)-amino]-piperidin-1-yl}-pentancarbonsäure-2-methoxyethylester
(18) 2-Methyl-2-phenyl-5-{4-[(4'-trifluormethyl-biphenyl-2-carbonyl)-amino]-piperidin-1-yl}-pentancarbonsäure-2-ethoxyethylester
(19) 2-Methyl-2-phenyl-5-{4-[(4'-trifluormethyl-biphenyl-2-carbonyl)-amino]-piperidin-1-yl}-pentäncarbonsäure-3-methoxypropylester
(20) 2-Methyl-2-phenyl-5-{4-[(4'-trifluormethyl-biphenyl-2-carbonyl)-amino]-piperidin-1-yl}-pentancarbonsäure-3-ethoxypropylester
(21) 2-Methyl-2-phenyl-5-{4-[(4'-trifluormethyl-biphenyl-2-carbonyl)-amino]-piperidin-1-yl}-pentancarbonsäure-2-aminoethylester
(22) 2-Methyl-2-phenyl-5-{4-[(4'-trifluormethyl-biphenyl-2-carbonyl)-amino]-piperidin-1-yl}-pentancarbonsäure-3-aminopropylester
(23) 2-Methyl-2-phenyl-5-{4-[(4'-trifluormethyl-biphenyl-2-carbonyl)-amino]-piperidin-1-yl}-pentancarbonsäure-2-methylaminoethylester
(24) 2-Methyl-2-phenyl-5-{4-[(4'-trifluormethyl-biphenyl-2-carbonyl)-amino]-piperidin-1-yl}-pentancarbonsäure-3-methylaminopropylester
(25) 2-Methyl-2-phenyl-5-{4-[(4'-trifluormethyl-biphenyl-2-carbonyl)-amino]-piperidin-1-yl}-pentancarbansäure-2-dimethylaminoethylester
(26) 2-Methyl-2-phenyl-5-{4-[(4'-trifluormethyl-biphenyl-2-carbonyl)-amino]-piperidin-1-yl}-pentancarbonsäure-3-dimethylaminopropylester
(27) 2-Methyl-2-phenyl-5-{4-[(4'-trifluormethyl-biphenyl-2-carbonyl)-amino]-piperidin-1-yl}-pentancarbonsäure-benzylester
(28) 2-Methyl-2-phenyl-5-{4-[(4'-trifluormethyl-biphenyl-2-carbonyl)-amino]-piperidin-1-yl}-pentancarbonsäure-pyridin-2-yl-methylester
(29) 2-Methyl-2-phenyl-5-{4-[(4'-trifluormethyl-biphenyl-2-carbonyl)-amino]-piperidin-1-yl}-pentancarbonsäure-pyridin-3-yl-methylester
(30) 2-Methyl-2-phenyl-5-{4-[(4'-trifluormethyl-biphenyl-2-carbonyl)-amino]-piperidin-1-yl}-pentancarbonsäure-pyridin-4-yl-methylester
(31) 2-Methyl-2-phenyl-5-{4-[(4'-trifluormethyl-biphenyl-2-carbonyl)-amino]-piperidin-1-yl}-pentancarbonsäure-pyrimidin-4-yl-methylester
(32) 2-Methyl-2-(4-fluor-phenyl)-5-{4-[(4'-crifluormethyl-biphenyl-2-carbonyl)-amino]-piperidin-1-yl}-pentancarbonsäuremethylester
(33) 2-Methyl-2-(4-fluor-phenyl)-5-{4-[(4'-trifluormethyl-biphenyl-2-carbonyl)-amino]-piperidin-1-yl}-pentancarbonsäureethylester
(34) 2-Methyl-2-(4-fluor-phenyl)-5-{4-[(4'-trifluormethyl-biphenyl-2-carbonyl)-amino]-piperidin-1-yl}-pentancarbonsäurepropylester
(35) 2-Methyl-2-(4-fluor-phenyl)-5-{4-[(4'-trifluormethyl-biphenyl-2-carbonyl)-amino]-piperidin-1-yl}-pentancarbonsäureisopropylester
(36) 2-Methyl-2-(4-fluor-phenyl)-5-{4-[(4'-trifluormethyl-biphenyl-2-carbonyl)-amino]-piperidin-1-yl}-pentancarbonsäuretert.butylester
(37) 2-Methyl-2-phenyl-5-{4-[(4'-methyl-biphenyl-2-carbonyl)-amino]-piperidin-1-yl}-pentancarbonsäure-methylester
(38) 2-Methyl-2-phenyl-5-{4-[(4'-methyl-biphenyl-2-carbonyl)amino]-piperidin-1-yl}-pentancarbonsäure-ethylester
(39) 2-Methyl-2-phenyl-5-{4-[(4'-methyl-biphenyl-2-carbonyl)amino]-piperidin-1-yl}-pentancarbonsäure-propylester
(40) 2-Methyl-2-phenyl-5-{4-[(4'-methyl-biphenyl-2-carbonyl)amino]-piperidin-1-yl}-pentancarbonsäure-isopropylester
(41) 2-Methyl-2-phenyl-5-{4-[(4'-methyl-biphenyl-2-carbonyl)-amino]-piperidin-1-yl}-pentancarbonsäure-tert.butylester
(42) 2-Methyl-2-phenyl-5-{4-[(4'-ethyl-biphenyl-2-carbonyl)-amino]-piperidin-1-yl}-pentancarbonsäure-methylester
(43) 2-Methyl-2-phenyl-5-{4-[(4'-ethyl-biphenyl-2-carbonyl)-amino]-piperidin-1-yl}-pentancarbonsäure-ethylester
(44) 2-Methyl-2-phenyl-5-{4-[(4'-ethyl-biphenyl-2-carbonyl)-amino]-piperidin-1-yl}-pentancarbonsäure-propylester
(45) 2-Methyl-2-phenyl-5-{4-[(4'-ethyl-biphenyl-2-carbonyl)amino]-piperidin-1-yl}-pentancarbonsäure-isopropylester
(46) 2-Methyl-2-phenyl-5-{4-[(4'-ethyl-biphenyl-2-carbonyl)amino]-piperidin-1-yl}-pentancarbonsäure-tert.butylester
(47) 2-Methyl-2-phenyl-5-{4-[(4'-fluor-biphenyl-2-carbonyl)amino]-piperidin-1-yl)}-pentancarbonsäure-methylester
(48) 2-Methyl-2-phenyl-5-{4-[(4'-fluor-biphenyl-2-carbonyl)amino]-piperidin-1-yl}-pentancarbonsäure-ethylester
(49) 2-Methyl-2-phenyl-5-{4-[(4'-fluor-biphenyl-2-carbonyl)amino]-piperidin-1-yl}-pentancarbonsäure-propylester
(50) 2-Methyl-2-phenyl-5-{4-[(4'-fluor-biphenyl-2-carbonyl)-amino]-piperidin-1-yl}-pentancarbonsäure-isopropylester
(51) 2-Methyl-2-phenyl-5-{4-[(4'-fluor-biphenyl-2-carbonyl)-amino]-piperidin-1-yl}-pentancarbonsäure-tert. butylester
(52) 2-Methyl-2-phenyl-5-{4-[(4'-chlor-biphenyl-2-carbonyl)-amino]-piperidin-1-yl}-pentancarbonsäure-methylester
(53) 2-Methyl-2-phenyl-5-{4-[(4'-chlor-biphenyl-2-carbonyl)amino]-piperidin-1-yl}-pentancarbonsäure-ethylester
(54) 2-Methyl-2-phenyl-5-{4-[(4'-chlor-biphenyl-2-carbonyl)amino]-piperidin-1-yl}-pentancarbonsäure-propylester
(55) 2-Methyl-2-phenyl-5-{4-[(4'-chlor-biphenyl-2-carbonyl)amino]-piperidin-1-yl}-pentancarbonsäure-isopropylester
(56) 2-Methyl-2-phenyl-5-{4-[(4'-chlor-biphenyl-2-carbonyl)amino]-piperidin-1-yl}-pentancarbonsäure-tert.butylester
(57) 2-Methyl-2-phenyl-5-{4-[(4'-brom-biphenyl-2-carbonyl)-amino]-piperidin-1-yl}-pentancarbonsäure-methylester
(58) 2-Methyl-2-phenyl-5-{4-[(4'-brom-biphenyl-2-carbonyl)-amino]-piperidin-1-yl}-pentancarbonsäure-ethylester
(59) 2-Methyl-2-phenyl-5-{4-[(4'-brom-biphenyl-2-carbonyl)-amino]-piperidin-1-yl}-pentancarbonsäure-propylester
(60) 2-Methyl-2-phenyl-5-{4-[(4'-brom-biphenyl-2-carbonyl)-amino]-piperidin-1-yl}-pentancarbonsäure-isopropylester
(61) 2-Methyl-2-phenyl-5-{4-[(4'-brom-biphenyl-2-carbonyl)-amino]-piperidin-1-yl}-pentancarbonsäure-tert.butylester
(62) 2-Methyl-2-phenyl-5-{4-[(4'-methoxy-biphenyl-2-carbonyl)-amino]-piperidin-1-yl}-pentancarbonsäure-methylester
(63) 2-Methyl-2-phenyl-5-{4-[(4'-methoxy-biphenyl-2-carbonyl)-amino]-piperidin-1-yl}-pentancarbonsäure-ethylester
(64). 2-Methyl-2-phenyl-5-{4-[(4'-methoxy-biphenyl-2-carbonyl)-amino]-piperidin-1-yl}-pentancarbonsäure-propylester
(65) 2-Methyl-2-phenyl-5-{4-[(4'-methoxy-biphenyl-2-carbonyl)-amino]-piperidin-1-yl}-pentancarbonsäure-isopropylester
(66) 2-Methyl-2-phenyl-5-{4-[(4'-methoxy-biphenyl-2-carbonyl)-amino]-piperidin-1-yl}-pentancarbonsäure-tert.butylester
(67) 2-Methyl-2-phenyl-5-{4-[(3'-methyl-biphenyl-2-carbonyl)-amino]-piperidin-1-yl}-pentancarbonsäure-methylester
(68) 2-Methyl-2-phenyl-5-{4-[(3'-methyl-biphenyl-2-carbonyl)-amino]-piperidin-1-yl}-pentancarbonsäure-ethylester
(69) 2-Methyl-2-phenyl-5-{4-[(3'-methyl-biphenyl-2-carbonyl)-amino]-piperidin-1-yl}-pentancarbonsäure-propylester
(70) 2-Methyl-2-phenyl-5-{4-[(3'-methyl-biphenyl-2-carbonyl)-amino]-piperidin-1-yl}-pentancarbonsäure-isopropylester
(71) 2-Methyl-2-phenyl-5-{4-[(3'-methyl-biphenyl-2-carbonyl)-amino]-piperidin-1-yl}-pentancarbonsäure-tert butylester
(72) 2-Methyl-2-phenyl-5-{4-[(3'-fluor-biphenyl-2-carbonyl)-amino]-piperidin-1-yl}-pentancarbonsäure-methylester
(73) 2-Methyl-2-phenyl-5-{4-[(3'-fluor-biphenyl-2-carbonyl)-amino]-piperidin-1-yl}-pentancarbonsäure-ethylester
(74) 2-Methyl-2-phenyl-5-{4-[(3'-fluor-biphenyl-2-carbonyl)-amino]-piperidin-1-yl}-pentancarbonsäure-propylester
(75) 2-Methyl-2-phenyl-5-{4-[(3'-fluor-biphenyl-2-carbonyl)-amino]-piperidin-1-yl}-pentancarbonsäure-isopropylester
.(76) 2-Methyl-2-phenyl-5-{4-[(3'-fluor-biphenyl-2-carbonyl)-amino]-piperidin-1-yl}-pentancarbonsäure-tert.butylester
(77) 2-Methyl-2-phenyl-5-{4-[(3'-chlor-biphenyl-2-carbonyl)amino]-piperidin-1-yl}-pentancarbonsäure-methylester
(78) 2-Methyl-2-phenyl-5-{4-[(3'-chlor-biphenyl-2-carbonyl)-amino]-piperidin-1-yl}-pentancarbonsäure-ethylester
(79) 2-Methyl-2-phenyl-5-{4-[(3'-chlor-biphenyl-2-carbonyl)-amino]-piperidin-1-yl}-pentancarbonsäure-propylester
(80) 2-Methyl-2-phenyl-5-{4-[(3'-chlor-biphenyl-2-carbonyl)-amino]-piperidin-1-yl}-pentancarbonsäure-isopropylester
(81) 2-Methyl-2-phenyl-5-{4-[(3'-chlor-biphenyl-2-carbonyl)-amino]-piperidin-1-yl}-pentancarbonsäure-tert.butylester
(82) 2-Methyl-2-phenyl-5-{4-[(4,4'-dichlor-biphenyl-2-carbonyl)-amino]-piperidin-1-yl}-pentancarbonsäure-methylester
(83) 2-Methyl-2-phenyl-5-{4-[(4,4'-dichlor-biphenyl-2-carbonyl)-amino]-piperidin-1-yl}-pentancarbonsäure-ethylester
(84) 2-Methyl-2-phenyl-5-{4-[(4,4'-dichlor-biphenyl-2-carbonyl)-amino]-piperidin-1-yl}-pentancarbonsäurepropylester
(85) 2-Methyl-2-phenyl-5-{4-[(4,4'-dichlor-biphenyl-2-carbonyl)-amino]-piperidin-1-yl}-pentancarbonsäure-isopropylester
(86) 2-Methyl-2-phenyl-5-{4-[(4,4'-dichlor-biphenyl-2-carbonyl)-amino]-piperidin-1-yl}-pentancarbonsäure-tert.butylester

### Beispiel 2

### 2-Ethyl-2-phenyl-5-{4-[(4'-trifluormethyl-biphenyl-2-carbonyl)-amino]-piperidin-1-yl}-pentancarbonsäure-methylester

### a. 2-Phenylbutancarbonsäure-methylester

15 g (0.091 Mol) 2-Phenylbutancarbonsäure werden in 150 ml methanolischer Salzsäure gelöst und 18 Stunden bei Raumtemperatur gerührt. Das Lösungsmittel wird entfernt und der Rückstand mit Essigsäureethylester und gesättigter Natriumhydrogencarbonatlösung extrahiert. Die organischen Phasen werden mit Wasser und gesättigter Kochsalzlösung extrahiert, über Magnesiumsulfat getrocknet und eingedampft.
Ausbeute: 14,4 g (88,8% der Theorie),
C₁₁H₁₄O₂ (M = 178,23)
Ber.: Molpeak (M+Na)⁺ = 201
Gef.: Molpeak (M+Na)⁺ = 201

### b. 5-Brom-2-ethyl-2-phenyl-pentacarbonsäure-methylester

Zu einer Lösung von 11.35 ml (0.081 Mol) Diisopropylamin in 200 ml wasserfreiem Tecrahydrofuran werden bei -30°C 15 g (0.081 Mol) n-Butyllithium als 2.5-molare Lösung in Hexan zugetropft und 10 Minuten bei -10°C gerührt. Bei -76°C werden 14.4 g (0.081 Mol) 2-Phenylbutancarbonsäure-methylester zugetropft und 30 Minuten bei dieser Temperatur gerührt. Anschließend werden 8.62 ml (0.085 Mol) 1,3-Dibrompropan zugesetzt, nach beendeter Zugabe das Kühlbad entfernt und 14 Stunden bei. Raumtemperatur gerührt. Die Reaktionslösung wird auf 1.2 1 Wasser gegossen und mit Diethylether extrahiert. Die organischen Phasen werden mit Wasser extrahiert, über Natriumsulfat getrocknet und das Lösungsmittel entfernt. Der Rückstand wird im Hochvakuum destilliert.
Ausbeute: 10.1 g (41.7 % der Theorie),
Siedepunkt: 127°C bei 0,22 mmbar

### c. 2-Ethyl-2-phenyl-5-{4-[(4'-trifluormethyl-biphenyl-2-carbonyl)-amino]-piperidin-1-yl}-pentancarbonsäure-methylester

0.2 g (0.574 mMol) 4'-Trifluormethyl-biphenyl-2-carbonsäurepiperidin-4-yl-amid, 0.15 g (0.5 mMol) 5-Brom-2-ethyl-2-phenyl-pentancarbonsäure-methylester und 0.083 g (0.6 mMol) Kaliumcarbonat werden in 10 ml Acetonitril gelöst. Es wird 8 Stunden bei 60°C und 14 Stunden bei Raumtemperatur gerührt. Im Anschluß wird das Reaktionsgemisch auf Wasser gegossen und mit Essigsäure-ethylester extrahiert. Die organische Phase wird über Natriumsulfat getrocknet und das Lösungsmittel am Rotationsverdampfer abdestilliert. Nach Säulenchromatographie an Kieselgel (Eluens: Dichlormethan/Methanol = 20:1) verbleiben farblose Kristalle.
Ausbeute: 0.172 g (52.9 % der Theorie),
C₃₃H₃₇F₃N₂O₃ (M = 566,67)
Schmelzpunkt: 135°C
Ber:: Molpeak (M+H)⁺ = 567
Gef.: Molpeak (M+H)⁺ = 567
Analog Beispiel 2 können folgende Verbindungen hergestellt werden:
(1) 2-Ethyl-2-phenyl-5-{4-[(4'-trifluormethyl-biphenyl-2-carbonyl)-amino]-piperidin-1-yl}-pentancarbonsäure-ethylester Hergestellt aus 4'-Trifluormethyl-biphenyl-2-carbonsäure-piperidin-4-ylamid und 5-Brom-2-ethyl-2-phenyl-pentancarbonsäureethylester.
   Ausbeute: 0,052 g (6 % der Theorie);
   C₃₄H₃₉F₃N₂O₃ (580,69)
   Schmelzpunkt: 110°C
   Ber.: Molpeak (M+H)⁺ = 581
   Gef.: Molpeak (M+H)⁺ = 581
(2) 2-Ethyl-2-phenyl-5-{4-[(4'-trifluormethyl-biphenyl-2-carbonyl)-amino]-piperidin-1-yl}-pentancarbonsäure-propylester
(3) 2-Ethyl-2-phenyl-5-{4-[(4'-trifluormethyl-biphenyl-2-carbonyl)-amino]-piperidin-1-yl}-pentancarbonsäure-butylester
(4) 2-Ethyl-2-phenyl-5-{4-[(4'-trifluormethyl-biphenyl-2-carbonyl)-amino]-piperidin-1-yl}-pentancarbonsäure-pentylester
(5) 2-Ethyl-2-phenyl-5-{4-[(4'-trifluormethyl-biphenyl-2-carbonyl)-amino]-piperidin-1-yl}-pentancarbonsäure-hexylester
(6) 2-Ethyl-2-phenyl-5-{4-[(4'-trifluormethyl-biphenyl-2-carbonyl)-amino]-piperidin-1-yl}-pentancarbonsäure-isopropylester Hergestellt aus 4'-Trifluormethyl-biphenyl-2-carbonsäure-piperidin-4-ylamid und 5-Brom-2-ethyl-2-phenyl-gentancarbonsäureisopropylester.
   Ausbeute: 0,153 g (16,8 % der Theorie),
   C₃₅H₄₁F₃N₂O₃ (594,72)
   Schmelzpunkt: 124-125°C
   Ber.: Molpeak (M+H)⁺ = 595
   Gef.: Molpeak (M+H)⁺ = 595
(7) 2-Ethyl-2-phenyl-5-{4-[(4'-trifluormethyl-biphenyl-2-carbonyl)-amino]-piperidin-1-yl}-pentancarbonsäure-tert.butylester
(8) 2-Ethyl-2-phenyl-5-{4-[(4'-trifluormethyl-biphenyl-2-carbonyl)-amino]-piperidin-1-yl}-pentancarbonsäure-isobutylester
(9) 2-Ethyl-2-phenyl-5-{4-[(4'-trifluormethyl-biphenyl-2-carbonyl)-amino]-piperidin-1-yl}-pentancarbonsaure-sec.butylester
(10) 2-Ethyl-2-phenyl-5-{4-[(4'-trifluormethyl-biphenyl-2-carbonyl)-amino]-piperidin-1-yl}-pentancarbonsäure-cyclopropylester
(11) 2-Ethyl-2-phenyl-5-{4-[(4'-trifluormethyl-biphenyl-2-carbonyl)-amino]-piperidin-1-yl}-pentancarbonsäure-cyclobutylester
(12) 2-Ethyl-2-phenyl-5-{4-[(4'-trifluormethyl-biphenyl-2-carbonyl)-amino]-piperidin-1-yl}-pentancarbonsäure-cyclopentylester
(13) 2-Ethyl-2-phenyl-5-{4-[(4'-trifluormethyl-biphenyl-2-carbonyl)-amino]-piperidin-1-yl}-pentancarbonsäure-cyclohexylester
(14) 2-Ethyl-2-phenyl-5-{4-[(4,'-trifluormethyl-biphenyl-2-carbonyl)-amino]-piperidin-1-yl}-pentancarbonsäure-cycloheptylester
(15) 2-Ethyl-2-phenyl-5-{4-[(4'-trifluormethyl-biphenyl-2-carbonyl)-amino]-piperidin-1-yl}-pentancarbonsäure-2-hydroxyethylester
(16) 2-Ethyl-2-phenyl-5-{4-[(4'-trifluormethyl-biphenyl-2-carbonyl)-amino]-piperidin-1-yl}-pentancarbonsäure-3-hydroxypropylester
(17) 2-Ethyl-2-phenyl-5-{4-[(4'-trifluormethyl-biphenyl-2-carbonyl)-amino]-piperidin-1-yl}-pentancarbonsäure-2-methoxyethylester
(18) 2-Ethyl-2-phenyl-5-{4-[(4'-trifluormethyl-biphenyl-2-carbonyl)-amino]-piperidin-1-yl}-pentancarbonsäure-2-ethoxyethylester
(19) 2-Ethyl-2-phenyl-5-{4-[(4'-trifluormethyl-biphenyl-2-carbonyl)-amino]-piperidin-1-yl}-pentancarbonsäure-3-methoxypropylester
(20) 2-Ethyl-2-phenyl-5-{4-[(4'-trifluormethyl-biphenyl-2-carbonyl)-amino]-piperidin-1-yl}-pentancarbonsäure-3-ethoxy-propylester
(21) 2-Ethyl-2-phenyl-5-{4-[(4'-trifluormethyl-biphenyl-2-carbonyl)-amino]-piperidin-1-yl}-pentancarbonsäure-2-amino-ethylester
(22) 2-Ethyl-2-phenyl-5-{4-[(4'-trifluormethyl-biphenyl-2-carbonyl)-amino]-piperidin-1-yl}-pentancarbonsäure-3-amino-propylester
(23) 2-Ethyl-2-phenyl-5-{4-[(4'-trifluormethyl-biphenyl-2-carbonyl)-amino]-piperidin-1-yl}-pentancarbonsäure-2-methylaminoethylester
(24) 2-Ethyl-2-phenyl-5-{4-[(4'-trifluormethyl-biphenyl-2-carbonyl)-amino]-piperidin-1-yl} -pentancarbonsäure-3-methylaminopropylester
(25) 2-Ethyl-2-phenyl-5-{4-[(4'-trifluormethyl-biphenyl-2-carbonyl)-amino]-piperidin-1-yl}-pentancarbonsäure-2-dimethylaminoethylester
(26) 2-Ethyl-2-phenyl-5-{4-[(4'-trifluormethyl-biphenyl-2-carbonyl)-amino]-piperidin-1-yl}-pentancarbonsäure-3-dimethylaminopropylester
(27) 2-Ethyl-2-phenyl-5-{4-[(4'-trifluormethyl-biphenyl-2-carbonyl)-amino]-piperidin-1-yl}-pentancarbonsäure-benzylester
(28) 2-Ethyl-2-phenyl-5-{4- [(4'-trifluormethyl-biphenyl-2-carbonyl)-amino]-piperidin-1-yl}-pentancarbonsäure-pyridin-2-ylmethylester
(29) 2-Ethyl-2-phenyl-5-{4-[(4'-trifluormethyl-biphenyl-2-carbonyl)-amino]-piperidin-1-yl}-pentancarbonsäure-pyridin-3-ylmethylester
(30) 2-Ethyl-2-phenyl-5-{4-[(4'-trifluormethyl-biphenyl-2-carbonyl)-amino]-piperidin-1-yl}-pentancarbonsäure-pyridin-4-ylmethylester
(31) 2-Ethyl-2-phenyl-5-{4-[(4'-trifluormethyl-biphenyl-2-carbonyl)-amino]-piperidin-1-yl}-pentancarbonsäure-pyrimidin-4-yl-methylester
(32) 2-Ethyl-2-(4-fluor-phenyl)-5-{4-[(4'-trifluormethyl-biphenyl-2-carbonyl)-amino]-piperidin-1-yl}-pentancarbonsäuremethylester
(33) 2-Ethyl-2-(4-fluor-phenyl)-5-{4-[(4'-trifluormethyl-biphenyl-2-carbonyl)-amino]-piperidin-1-yl}-pentancarbonsäureethylester
(34) 2-Ethyl-2-(4-fluor-phenyl)-5-{4-[(4'-trifluormethyl-biphenyl-2-carbonyl)-amino]-piperidin-1-yl}-pentancarbonsäurepropylester
(35) 2-Ethyl-2-(4-fluor-phenyl)-5-{4-[(4'-trifluormethyl-biphenyl-2-carbonyl)-amino]-piperidin-1-yl}-pentancarbonsäureisopropylester
(36) 2-Ethyl-2-(4-fluor-phenyl)-5-{4-[(4'-trifluormethyl-biphenyl-2-carbonyl)-amino]-piperidin-1-yl}-pentancarbonsäüretert.butylester
(37) 2,2-Diphenyl-6-{4-[(4'-trifluormethyl-biphenyl-2-carbonyl)-amino]-piperidin-1-yl}-pentancarbonsäure-methylester
(38) 2,2-Diphenyl-6-{4-[(4'-trifluormethyl-biphenyl-2-carbonyl)-amino]-piperidin-1-yl}-pentancarbonsäure-echylester
(39) 2, 2-Diphenyl-6-{4-[(4'-trifluormethyl-biphenyl-2-carbonyl)-amino]-piperidin-1-yl}-pentancarbonsäure-propylester
(40) 2,2-Diphenyl-6-{4-[(4'-trifluormethyl-biphenyl-2-carbonyl)-amino]-piperidin-1-yl}-pentancarbonsäure-isopropylester
(41) 2,2-Diphenyl-6-{4-[(4'-trifluormethyl-biphenyl-2-carbonyl)-amino]-piperidin-1-yl}-pentancarbonsäure-tert.butylester
(42) 2 -Ethyl-2-phenyl-5-{4-[(4'-methyl-biphenyl-2-carbonyl)amino]-piperidin-1-yl}-pentancarbonsäure-methylester-hydrobromid
   Hergestellt aus 4'-Methyl-biphenyl-2-carbonsäure-piperidin-4-ylamid und 5-Brom-2-ethyl-2-phenyl-pentancarbonsäuremethylester.
   Ausbeute: 0,49 g (61 % der Theorie),
   C₃₃H₄₀N₂O₃ (593,61)
   Schmelzpunkt: 160°C
   Ber.: Molpeak (M+H)⁺ = 513
   Gef.: Molpeak (M+H)⁺ = 513
(43) 2-Ethyl-2-phenyl-5-{4-[(4'-methyl-biphenyl-2-carbonyl)-amino]-piperidin-1-yl}-pentancarbonsäure-ethylester
(44) 2-Ethyl-2-phenyl-5-{4-[(4'-methyl-biphenyl-2-carbonyl)-amino]-piperidin-1-yl}-pentancarbonsäure-propylester
(45) 2-Ethyl-2-phenyl-5-{4-[(4'-methyl-biphenyl-2-carbonyl)amino]-piperidin-1-yl}-pentancarbonsäure-isopropylester
(46) 2-Ethyl-2-phenyl-5-{4-[(4'-methyl-biphenyl-2-carbonyl)-amino]-piperidin-1-yl}-pentancarbonsäure-tert.butylester
(47) 2-Ethyl-2-phenyl-5-{4-[(4'-ethyl-biphenyl-2-carbonyl)-amino]-piperidin-1-yl}-pentancarbonsäure-methylester
(48) 2-Ethyl-2-phenyl-5-{4-[(4'-ethyl-biphenyl-2-carbonyl)-amino]-piperidin-1-yl}-pentancarbonsäure-ethylester
(49) 2-Ethyl-2-phenyl-5-{4-[(4'-ethyl-biphenyl-2-carbonyl)-amino]-piperidin-1-yl}-pentancarbonsäure-propylester
(50) 2-Ethyl-2-phenyl-5-{4-[(4'-ethyl-biphenyl-2-carbonyl)-amino]-piperidin-1-yl}-pentancarbonsäure-isopropylester
(51) 2-Ethyl-2-phenyl-5-{4-[(4'-ethyl-biphenyl-2-carbonyl)-amino]-piperidin-1-yl}-pentancarbonsäure-tert butylester
(52) 2-Ethyl-2-phenyl-5-{4-[(4'-fluor-biphenyl-2-carbonyl)-amino]-piperidin-1-yl}-pentancarbonsäure-methylester
(53) 2-Ethyl-2-phenyl-5-{4-[(4'-fluor-biphenyl-2-carbonyl)-amino]-piperidin-1-yl}-pentancarbonsäure-ethylester
(54) 2-Ethyl-2-phenyl-5-{4-[(4'-fluor-biphenyl-2-carbonyl)amino]-piperidin-1-yl}-pentancarbonsäure-propylester
(55) 2-Ethyl-2-phenyl-5-{4-[(4'-fluor-biphenyl-2-carbonyl)-amino]-piperidin-1-yl}-pentancarbonsäure-isopropylester
(56) 2-Ethyl-2-phenyl-5-{4-[(4'-fluor-biphenyl-2-carbonyl)amino]-piperidin-1-yl}-pentancarbonsäure-tert.butylester
(57) 2-Ethyl-2-phenyl-5-{4-[(4'-chlor-biphenyl-2-carbonyl)-amino]-piperidin-1-yl}-pentancarbonsäure-methylester
(58) 2-Ethyl-2-phenyl-5-{4-[(4'-chlor-biphenyl-2-carbonyl)amino]-piperidin-1-yl}-pentancarbonsäure-ethylester
(59) 2-Ethyl-2-phenyl-5-{4-[(4-chlor-biphenyl-2-carbonyl)-amino]-piperidin-1-yl}-pentancarbonsäure-propylester
(60) 2-Ethyl-2-phenyl-5-{4-[(4'-chlor-biphenyl-2-carbonyl)-amino]-piperidin-1-yl}-pentancarbonsäure-isopropylester
(61) 2-Ethyl-2-phenyl-5-{4-[(4'-chlor-biphenyl-2-carbonyl)-amino]-piperidin-1-yl}-pentancarbonsäure-tert.butylester
(62) 2-Ethyl-2-phenyl-5-{4-[(4'-brom-biphenyl-2-carbonyl)amino]-piperidin-1-yl}-pentancarbonsäure-methylester
(63) 2-Ethyl-2-phenyl-5-{4-[(4'-brom-biphenyl-2-carbonyl)-amino]-piperidin-1-yl}-pentancarbonsäure-ethylester
(64) 2-Ethyl-2-phenyl-5-{4-[(4'-brom-biphenyl-2-carbonyl)amino]-piperidin-1-yl}-pentancarbonsäure-propylester
(65) 2-Ethyl-2-phenyl-5-{4-[(4'-brom-biphenyl-2-carbonyl)amino]-piperidin-1-yl}-pentancarbonsäure-isopropylester
(66) 2-Ethyl-2-phenyl-5-{4-[(4'-brom-biphenyl-2-carbonyl)amino]-piperidin-1-yl}-pentancarbonsäure-tert.butylester
(67) 2-Ethyl-2-phenyl-5-{4-[(4'-methoxy-biphenyl-2-carbonyl)-amino]-piperidin-1-yl}-pentancarbonsäure-methylester
(68) 2-Ethyl-2-phenyl-5-{4-[(4'-methoxy-biphenyl-2-cärbonyl)-amino]-piperidin-1-yl}-pentancarbonsäure-ethylester
(69) 2-Ethyl-2-phenyl-5-{4-[(4'-methoxy-biphenyl-2-carbonyl)-amino]-piperidin-1-yl}-pentancarbonsäure-propylester
(70) 2-Ethyl-2-phenyl-5-{4-[(4'-methoxy-biphenyl-2-carbonyl)-amino]-piperidin-1-yl}-pentancarbonsäure-isopropylester
(71) 2-Ethyl-2-phenyl-5-{4-[(4'-methoxy-biphenyl-2-carbonyl)-amino]-piperidin-1-yl}-pentancarbonsäure-tert.butylester
(72) 2-Ethyl-2-phenyl-5-{4-[(3'-methyl-biphenyl-2-carbonyl)-amino]-piperidin-1-yl}-pentancarbonsäure-methylester
(73) 2-Ethyl-2-phenyl-5-{4-[(3'-methyl-biphenyl-2-carbonyl)amino]-piperidin-1-yl}-pentancarbonsäure-ethylester
(74) 2-Ethyl-2-phenyl-5-{4-[(3'-methyl-biphenyl-2-carbonyl)amino]-piperidin-1-yl}-1-pentancarbonsäure-propylester
(75) 2-Ethyl-2-phenyl-5-{4-[(3'-methyl-biphenyl-2-carbonyl)-amino]-piperidin-1-yl}-pentancarbonsäure-isopropylester
(76) 2-Ethyl-2-phenyl-5-{4-[(3'-methyl-biphenyl-2-carbonyl)-amino]-piperidin-1-yl}-pentancarbonsäure-tert.butylester
(77) 2-Ethyl-2-phenyl-5-{4-[(3'-fluor-biphenyl-2-carbonyl)amino]-piperidin-1-yl}-pentancarbonsäure-methylester
(78) 2-Ethyl-2-phenyl-5-{4-[(3'-fluor-biphenyl-2-carbonyl)amino]-piperidin-1-yl}-pentancarbonsäure-ethylester
(79) 2-Ethyl-2-phenyl-5-{4-[(3'-fluor-biphenyl-2-carbonyl)-amino]-piperidin-1-yl}-pentancarbonsäure-propylester
(80) 2-Ethyl-2-phenyl-5-{4-[(3'-fluor-biphenyl-2-carbonyl)-amino]-piperidin-1-yl}-pentancarbonsäure-isopropylester
(81) 2-Ethyl-2-phenyl-5-{4-[(3'-fluor-biphenyl-2-carbonyl)amino]-piperidin-1-yl}-pentancarbonsäure-tert.butylester
(82) 2-Ethyl-2-phenyl-5-{4-[(3'-chlor-biphenyl-2'-carbonyl)-amino]-piperidin-1-yl}-pentancarbonsäure-methylester
(83) 2-Ethyl-2-phenyl-5-{4-[(3'-chlor-biphenyl-2-carbonyl)amino]-piperidin-1-yl}-pentancarbonsäure-ethylester
(84) 2-Ethyl-2-phenyl-5-{4-[(3'-chlor-biphenyl-2-carbonyl)amino]-piperidin-1-yl}-pentancarbonsäure-propylester
(85) 2-Ethyl-2-phenyl-5-{4-[(3'-chlor-biphenyl-2-carbonyl)-amino]-piperidin-1-yl}-pentancarbonsäure-isopropylester
(86) 2-Ethyl-2-phenyl-5-{4-[(3'-chlor-biphenyl-2-carbonyl)-amino]-piperidin-1-yl}-pentancarbonsäure-tert.butylester
(87) 2-Ethyl-2-phenyl-5,-{4-[(4,4'-dichlor-biphenyl-2-carbonyl)-amino]-piperidin-1-yl}-pentancarbonsäure-methylester
(88) 2-Ethyl-2-phenyl-5-{4-[(4,4'-dichlor-biphenyl-2-carbonyl)-amino]-piperidin-1-yl}-pentancarbonsäure-ethylester
(89) 2-Ethyl-2-phenyl-5-{4-[(4,4'-dichlor-biphenyl-2-carbonyl)-amino]-piperidin-1-yl}-pentancarbonsäure-propylester
(90) 2-Ethyl-2-phenyl-5-{4-[(4,4'-dichlor-biphenyl-2-carbonyl)-amino]-piperidin-1-yl}-pentancarbonsäure-isopropylester
(91) 2-Ethyl-2-phenyl-5-{4-[(4,4'-dichlor-biphenyl-2-carbonyl)-amino]-piperidin-1-yl}-pentancarbonsäure-tert.butylester

### Beispiel 3

### 2-Methyl-2-phenyl-5-{4-[(Biphenyl-2-carbonyl)-amino]-piperidin-1-yl}-pentancarbonsäure-methylester

### a. Biphenyl-2-carbonsäurechlorid

Zu einer Suspension von 3 g (0.015 Mol) ) Biphenyl-2-carbonsäure in 100 ml Dichlormethan und 0.3 ml Dimethylformamid werden bei 0°C 1.54 ml (0.018 Mol) Oxalylchlorid zugetropft. Nach Beendigung der Reaktion wird das Reaktionsgemisch durch Destillation eingeengt und das Biphenyl-2-carbonsäurechlorid als Rohprodukt weiter umgesetzt.
Ausbeute: 3 , 1 g (95,4 % der Theorie ) .

### b. Biphenyl-2-carbonsäure-(1-benzyl-piperidin-4-yl)-amid

Zu einer Lösung von 2.65 ml (0.013 Mol) 4-Amino-1-benzyl-piperidin und 6 ml (0.043 Mol) Triethylamin in 100 ml Dichlormethan werden 3 g (0.014 Mol) 4'-Trifluormethyl-biphenyl-2-carbonsäurechlorid, gelöst in 30 ml Dichlormethan, bei 15 bis -20°C zugetropft . Es wird 14 Stunden bei Raumtemperatur gerührt.. Die Reaktionlösung wird mit einer Mischung aus. 1-molaren Natriumhydrogencarbonatlösung und 1N Natronlauge extrahiert. Die organische Phase wird über Natriumsulfat getrocknet und das Lösungsmittel entfernt. Der Rückstand wird aus 650 ml Ethanol umkristallisiert.
Ausbeute: 2.93 g (56.5 % der Theorie),
Schmelzpunkt: 144-145°C

### c. Biphenyl-2-carbonsäure-piperidin-4-ylamid-dihydrochlorid

3.2 g (0.009 Mol) Biphenyl -2-carbonsäuer- (1-benzyl-piperidin-4-yl)-amid werden in 80 ml Ethanol gelöst, mit 0.7 g Palladiumhydroxid (20%ig auf Kohle) versetzt und in einer Parr-Apperatur in einer Wasserstoffatmosphäre (50 psi) fünf Stunden bei 50°C gerührt . Anschließend wird der Katalysator abfiltriert, der Rückstand eingeengt und in Essigsäureethylester gelöst. Nach Zusatz von ethanolischer Salzsäure wird der Niederschlag abfiltriert und bei 100°C im Umlufttrockenschrank getrocknet.
Ausbeute: 2.4 g (75.5 % der Theorie),
Schmelzpunkt: > 250°C
C₁₈H₂₀N₂O (M = 281,38)
Ber.: Molpeak (M+H)⁺ = 281
Gef.: Molpeak (M+H)⁺ = 281

### d. 2-Methyl-2-phenyl-5-{4-[(Biphenyl-2-carbonyl)-amino]-piperidin-1-yl}-pentancarbonsäure-methylester

Eine Lösung von 0.634 g (0.002 Mol) Biphenyl-2-carbonsäure-piperidin-4-ylamid, 0.5 g (1.75 mMol) 5-Brom-2-methyl-2-phenyl-pentancarbonsäuremethylester und 1 ml (0.007 Mol) Triethylamiri werden in 3 ml Dimethylformamid gelöst. Es wird 14 Stunden bei 80°C gerührt. Im Anschluß wird das Reaktionsgemisch auf Wasser gegossen und mit Essigsäureethylester extrahiert. Die organische Phase wird über Natriumsulfat getrocknet. Nach Säulenchromatographie an Kieselgel (Eluens: Dichlormethan/Methanol = 10:1) verbleiben farblose Kristalle. Ausbeute: 0.12 g (12.4 % der Theorie),
C₃₁H₃₆N₂O₃ (M = 484,64)
Schmelzpunkt: 96°C
Ber.: Molpeak (M+H)⁺ = 485
Gef.: Molpeak (M+H)⁺ = 485

Analog Beispiel 3 können folgende Verbindungen hergestellt werden:
(1) 2-Methyl-2-phenyl-5-{4-[(Biphenyl-2-carbonyl)-amino]-piperidin-1-yl}-pentancarbonsäure-ethylester
(2) 2-Methyl-2-phenyl-5-{4-[(Biphenyl-2-carbonyl)-amino]-piperidin-1-yl}-pentancarbonsäure-propylester
(3) 2-Methyl-2-phenyl-5-{4-[(Biphenyl-2-carbonyl)-amino]-piperidin-1-yl}-pentancarbonsäure-isopropylester
(4) 2-Ethyl-2-phenyl-5-{4-[(Biphenyl-2-carbonyl)-amino]-piperidin-1-yl}-pentancarbonsäure-methylester
(5) 2-Ethyl-2-phenyl-5-{4-[(Biphenyl-2-carbonyl)-amino]-piperidin-1-yl}-pentancarbonsäure-ethylester
(6) 2-Ethyl-2-phenyl-5-{4-[(Biphenyl-2-carbonyl)-amino]-piperidin-1-yl}-pentancarbonsäure-propylester
(7) 2-Ethyl-2-phenyl-5-{4-[(Biphenyl-2-carbonyl)-amino]-piperidin-1-yl}-pentancarbonsäure-isopropylester

### Beispiel 4

### 2-Methyl-2-phenyl-5-{4-[(4'-trifluormethyl-biphenyl-2-carbonyl)-amino]-piperidin-1-yl}-pentancarbonsäure

Eine Suspension von 1.5 g (0,0027 Mol) 2-Methyl-2-phenyl-5-{4-[(4'-trifluormethyl-biphenyl-2-carbonyl)-amino]-piperidin-1-yl}-pentancarbonsäure-methylester in 60 ml 6N Salzsäure wird 25 Stunden zum Rückfluß erhitzt. Nach dem Erkalten wird der Niederschlag abfiltriert und mit Wasser und Essigsäure-ethylester gewaschen.
Ausbeute: 1.1 g (75.8 % der Theorie),
C₃₁H₃₃F₃N₂O₃ (M = 538, 61)
Schmelzpunkt: 203-205°C
Ber.: Molpeak (M+H)⁺ = 538
Gef.: Molpeak (M+H)⁺ = 538

Analog Beispiel 4 können folgende Verbindungen hergestellt werden:
(1) 2-Methyl-2-phenyl-5-{4-[(4'-methyl-biphenyl-2-carbonyl)-amino]-piperidin-1-yl}-pentancarbonsäure
(2) 2-Methyl-2-phenyl-5-{4-[(4'-chlor-biphenyl-2-carbonyl)-amino]-piperidin-1-yl}-pentancarbonsäure
(3) 2-Methyl-2-phenyl-5-{4-[(4'-methoxy-biphenyl-2-carbonyl)-amino]-piperidin-1-yl}-pentancarbonsäure
(4) 2-Ethyl-2-phenyl-5-{4-[(4'-trifluormethyl-biphenyl-2-carbonyl)-amino]-piperidin-1-yl}-pentancarbonsäure
(5) 2-Ethyl-2-phenyl-5-{4-[(4'-methyl-biphenyl-2-carbonyl)-amino]-piperidin-1-yl}-pentancarbonsäure
(6) 2-Ethyl-2-phenyl-5-{4-[(4'-chlor-biphenyl-2-carbonyl)-amino]-piperidin-1-yl}-pentancarbonsäure
(7) 2-Ethyl-2-phenyl-5-{4-[(4'-methoxy-biphenyl-2-carbonyl)-amino]-piperidin-1-yl}-pentancarbonsäure

### Beispiel 5

### 2-Methyl-2-phenyl-5-{4-[(4'-trifluormethyl-biphenyl-2-carbonyl)-N-methyl-amino]-piperidin-1-yl}-pentancarbonsäure-methylester

Zu einer Lösung von 0.4 g (0.724 mMol) 2-Methyl-2-phenyl-5-{4-[(4'-trifluormethyl-biphenyl-2-carbonyl)-amino]-piperidin-1-yl}-pentancarbonsäure-methylester in 5 ml Dimethylformamid werden bei Raumtemperatur 0.095 g (0.803 mMol) Natriumhydrid (55 bis 65%ig in Öl) gegeben und eine Stunde gerührt. Anschließend werden 0.05 ml (0,803 mMol) Methyljodid zugesetzt und 14 Stunden gerührt. Die Reaktionslösung wird auf Wasser gegossen, mit Essigsäure-ethylester extrahiert, und die organische Phase über Natriumsulfat getrocknet. Die Reinigung erfolgt durch Säulenchromatographie an Kieselgel (Eluens: Dichlormethan/Methanol = 5:1).
Ausbeute: 0,04 g (10 % der Theorie),
C₃₃H₃₇F₃N₂O₃ (M = 566,67)
Ber.: Molpeak (M+H)⁺ = 567
Gef.: Molpeak (M+H)⁺ = 567

Analog Beispiel 5 können folgende Verbindungen hergestellt werden:
(1) 2-Methyl-2-phenyl-5-{4-[(4'-trifluormethyl-biphenyl-2-carbonyl)-N-methyl-amino]-piperidin-1-yl}-pentancarbonsäureethylester
(2) 2-Methyl-2-phenyl-5-{4-[(4'-trifluormethyl-biphenyl-2-carbonyl)-N-methyl-amino]-piperidin-1-yl}-pentancarbonsäure-propylester
(3) 2-Methyl-2-phenyl-5-{4-[(4'-trifluormethyl-biphenyl-2-carbonyl)-N-methyl-amino]-piperidin-1-yl}-pentancarbonsäure-isopropylester
(4) 2-Methyl-2-phenyl-5-{4-[(4'-trifluormethyl-biphenyl-2-carbonyl)-N-methyl-amino]-piperidin-1-yl}-pentancarbonsäuretert.butylester
(5) 2-Methyl-2-phenyl-5-{4-[(4'-trifluormethyl-biphenyl-2-carbonyl)-N-ethyl-amino]-piperidin-1-yl}-pentancarbonsäure-methylester
(6) 2-Methyl-2-phenyl-5-{4-[(4'-trifluormethyl-biphenyl-2-carbonyl)-N-ethyl-amino]-piperidin-1-yl}-pentancarbonsäure-ethylester
(7) 2-Methyl-2-phenyl-5-{4-[(4'-trifluormethyl-biphenyl-2-carbonyl)-N-ethyl-amino]-piperidin-1-yl}-pentancarbonsäure-propylester
(8) 2-Methyl-2-phenyl-5-{4-[(4'-trifluormethyl-biphenyl-2-carbonyl)-N-ethyl-amino]-piperidin-1-yl}-pentancarbonsäure-isopropylester
(9) 2-Methyl-2-phenyl-5-{4-[(4'-trifluormethyl-biphenyl-2-carbonyl)-N-ethyl-amino]-piperidin-1-yl}-pentancarbonsäure-tert.butylester
(10) 2-Ethyl-2-phenyl-5-{4-[(4'-trifluormethyl-biphenyl-2-carbonyl)-N-methyl-amino]-piperidin-1-yl}-pentancarbonsäure-methylester
(11) 2-Ethyl-2-phenyl-5-{4-[(4'-trifluormethyl-biphenyl-2-carbonyl)-N-methyl-amino]-piperidin-1-yl}-pentancarbonsäureethylester
(12) 2-Ethyl-2-phenyl-5-{4-[(4'-trifluormethyl-biphenyl-2-carbonyl)-N-methyl-amino]-piperidin-1-yl}-pentancarbonsäure-propylester
(13) 2-Ethyl-2-phenyl-5-{4-[(4'-trifluormethyl-biphenyl-2-carbonyl)-N-methyl-amino]-piperidin-1-yl}-pentancarbonsäure-isopropylester
(14) 2-Ethyl-2-phenyl-5-{4-[(4'-trifluormethyl-biphenyl-2-carbonyl)-N-methyl-amino]-piperidin-1-yl}-pentancarbonsäuretert.butylester
(15) 2-Ethyl-2-phenyl-5-{4-[(4'-trifluormethyl-biphenyl-2-carbonyl)-N-ethyl-amino]-piperidin-1-yl}-pentancarbonsäure-methylester
(16) 2-Ethyl-2-phenyl-5-{4-[(4'-trifluormethyl-biphenyl-2-carbonyl)-N-ethyl-amino]-piperidin-1-yl}-pentancarbonsäure-ethyl-ester
(17) 2-Ethyl-2-phenyl-5-{4-[(4'-trifluormethyl-biphenyl-2-carbonyl)-N-ethyl-amino]-piperidin-1-yl}-pentancarbonsäure-propylester
(18) 2-Ethyl-2-phenyl-5-{4-[(4'-trifluormethyl-biphenyl-2-carbonyl)-N-ethyl-amino]-piperidin-1-yl}-pentancarbonsäure-isopropylester
(19) 2-Ethyl-2-phenyl-5-{4-[(4'-trifluormethyl-biphenyl-2-carbonyl)-N-ethyl-amino]-piperidin-1-yl}-pentancarbonsäure-tert.butylester

### Beispiel 6

### 2-Methyl-2-phenyl-6-{4-[(4'-trifluormethyl-biphenyl-2-carbonyl)-amino]-piperidin-1-yl}-hexancarbonsäure-methylester

### a. 6-Brom-2-ethyl-2-phenyl-hexancarbonsäure-methylester

Zu einer Lösung von 14 ml (0.1 Mol) Diisopropylamin in 150 ml wasserfreiem Tetrahydrofuran werden bei -30°C. 40 ml (0.1 Mol) n-Butyllithium als 2.5 molare Lösung in Hexan zugetropft und 10 Minuten bei -10°C gerührt. Bei -76°C werden 16.4 g (0.1 Mol) 2-Phenylbutancarbonsäure-methylester zugetropft und 30 Minuten bei dieser Temperatur gerührt. Anschließend werden 12.12 ml (0.101 Mol) 1, 3-Dibrombutan zugesetzt, nach beendeter Zugabe das Kühlbad entfernt und 14 Stunden bei Raumtemperatur gerührt.. Die Reaktionslösung wird auf 1.2 1 Wasser gegossen und mit Diethylether extrahiert. Die organischen Phasen werden mit Wasser extrahiert, über Natriumsulfat getrocknet und das Lösungsmittel entfernt. Der Rückstand wird im Hochvakuum destilliert.
Ausbeute: 15.8 g (52.8 % der Theorie),
Siedepunkt: 100-117°C bei 0, 17. mmbar
C₁₄H₁₉BrO₂ (M = 299,21)
Ber.: Molpeak (M+Na)⁺ = 321/23
Gef.: Molpeak (M+Na)⁺ = 321/23

### b. 2-Methyl-2-phenyl-6-{4-[(4'-trifluormethyl-biphenyl-2-carbonyl)-amino]-piperidin-1-yl}-hexancarbonsäure-methylester

0.2 g (0.574 mMol) 4'-Trifluormethyl-biphenyl-2-carbonsäurepiperidin-4-yl-amid, 0.15 g (0.5 mMol) 6-Brom-2-methyl-2-phenyl-hexancarbonsäure-methyl ester und 0.083, g (0.6 mMol) Kaliumcarbonat werden in 10 ml Acetonitril gelöst. Es wird 8 Stunden bei 60°C und 14 Stunden bei Raumtemperatur gerührt. Im Anschluß wird das Reaktionsgemisch auf Wasser gegossen und mit Essigsäure-ethylester extrahiert. Die organische Phase wird über Natriumsulfat getrocknet und das Lösungsmittel am Rotationsverdampfer abdestilliert. Nach Säulenchromatographie an Kieselgel (Eluens: Dichlormethan/Methanol = 20:1) verbleibt ein hochviskoses Öl.
Ausbeute: 0.133 g (40.9 % der Theorie),
C₃₃H₃₇F₃N₂O₃ (M = 566,67)
Ber.: Molpeak (M+H)⁺ = 567
Gef.: Molpeak (M+H) ⁺ = 567

Analog Beispiel 6 können folgende Verbindungen hergestellt werden:
(1) 2-Methyl-2-phenyl-6-{4-[(4'-trifluormethyl-biphenyl-2-carbonyl)-amino]-piperidin-1-yl}-hexancarbonsäure-ethylester
(2) 2-Methyl-2-phenyl-6-{4-[(4'-trifluormethyl-biphenyl-2-carbonyl)-amino]-piperidin-1-yl}-hexancarbonsäure-propylester
(3) 2-Methyl-2-phenyl-6-{4-[(4'-trifluormethyl-biphenyl-2-carbonyl)-amino]-piperidin-1-yl}-hexancarbonsäure-isopropylester
(4) 2-Methyl-2-phenyl-6-{4-[(4'-trifluormethyl-biphenyl-2-carbonyl)-amino]-piperidin-1-yl}-hexancarbonsäure-tert.butylester
(5) 2-Ethyl-2-phenyl-6-{4-[(4'-trifluormethyl-biphenyl-2-carbonyl)-amino]-piperidin-1-yl}-hexancarbonsäure-methylester
(6) 2-Ethyl-2-phenyl-6-{4-[(4'-trifluormethyl-biphenyl-2-carbonyl)-amino]-piperidin-1-yl}-hexancarbonsäure-ethylester
(7) 2-Ethyl-2-phenyl-6-{4-[(4'-trifluormethyl-biphenyl-2-carbonyl)-amino]-piperidin-1-yl}-hexancarbonsäure-propylester
(8) 2-Ethyl-2-phenyl-6-{4-[(4'-trifluormethyl-biphenyl-2-carbonyl)-amino]-piperidin-1-yl}-hexancarbonsäure-isopropylester
(9) 2-Ethyl-2-phenyl-6-{4-[(4'-trifluormethyl-biphenyl-2-carbonyl)-amino]-piperidin-1-yl}-hexancarbonsäure-tert.butylester
(10) 2-Methyl-2-phenyl-4-{4-['(4'-trifluormethyl-biphenyl-2-carbonyl)-amino]-piperidin-1-yl}-butancarbonsäure-methylester
(11) 2-Methyl-2-phenyl-4-{4-[(4'-trifluormethyl-biphenyl-2-carbonyl)-amino]-piperidin-1-yl}-butancarbonsäure-ethylester
(12) 2-Methyl-2-phenyl-4-{4-[(4'-trifluormethyl-biphenyl-2-carbonyl)-amino]-piperidin-1-yl}-butancarbonsäure-propylester
(13) 2-Methyl-2-phenyl-4-{4-[(4'-trifluormethyl-biphenyl-2-carbonyl)-amino]-piperidin-1-yl}-butancarbonsäure-isopropylester
(14) 2-Methyl-2-phenyl-4-{4-[(4'-trifluormethyl-biphenyl-2-carbonyl)-amino]-piperidin-1-yl}-butancarbonsäure-tert.butylester
(15) 2-Ethyl-2-phenyl-4-{4-[(4'-trifluormethyl-biphenyl-2-carbonyl)-amino]-piperidin-1-yl}-butancarbonsäure-methylester
(16) 2-Ethyl-2-phenyl-4-{4-[(4'-trifluormethyl-biphenyl-2-carbonyl)-amino]-piperidin-1-yl}-butancarbonsäure-ethylester
(17) 2-Ethyl-2-phenyl-4-{4-[(4'-trifluormethyl-biphenyl-2-carbonyl)-amino]-piperidin-1-yl}-butancarbonsäure-propylester
(18) 2-Ethyl-2-phenyl-4-{4-[(4'-trifluormethyl-biphenyl-2-carbonyl)-amino]-piperidin-1-yl}-butancarbonsäure-isopropylester
(19) 2-Ethyl-2-phenyl-4-{4-[(4'-trifluormethyl-biphenyl-2-carbonyl)-amino]-piperidin-1-yl}-butancarbonsäure-tert.butylester
(20) 2-Methyl-2-phenyl-3-{4-[(4'-trifluormethyl-biphenyl-2-carbonyl)-amino]-piperidin-1-yl}-propancarbonsäure-methylester
(21) 2-Methyl-2-phenyl-3-{4-[(4'-trifluormethyl-biphenyl-2-carbonyl)-amino]-piperidin-1-yl}-propancarbonsäure-ethylester
(22) -Methyl-2-phenyl-3-{4-[(4'-trifluormethyl-biphenyl-2-carbonyl)-amino]-piperidin-1-yl}-propancarbonsäure-propylester
(23) 2-Methyl-2-phenyl-3-{4-[(4'-trifluormethyl-biphenyl-2-carbonyl)-amino]-piperidin-1-yl}-propancarbonsäure-isopropylester
(24) 2-Methyl-2-phenyl-3-{4-[(4'-trifluormethyl-biphenyl-2-carbonyl)-amino]-piperidin-1-yl}-propancarbonsäure-tert.butylester
(25) 2-Ethyl-2-phenyl-3-{4-[(4'-trifluormethyl-biphenyl-2-carbonyl)-amino]-piperidin-1-yl}-propancarbonsäure-methylester
(26) 2-Ethyl-2-phenyl-3-{4-[(4'-trifluormethyl-biphenyl-2-carbonyl)-amino]-piperidin-1-yl}-propancarbonsäure-ethylester
(27) 2-Ethyl-2-phenyl-3-{4-[(4'-trifluormethyl-biphenyl-2-carbonyl)-amino]-piperidin-1-yl}-propancarbonsäure-propylester
(28) 2-Ethyl-2-phenyl-3-{4-[(4'-trifluormethyl-bipheny-2-carbonyl)-amino]-piperidin-1-yl}-propancarbonsäure-isopropylester
(29) 2-Ethyl-2-phenyl-3-{4-[(4'-trifluormethyl-biphenyl-2-carbonyl)-amino]-piperidin-1-yl}-propancarbonsäure-tert.butylester

### Beispiel 7

### Tabletten mit 5 mg Wirkstoff pro Tablette

### Zusammensetzung:

| | |
|---|---|
| Wirkstoff | 5,0 mg |
| Lactose-monohydrat | 70,8 mg |
| Mikrokristalline Cellulose | 40,0 mg |
| Carboxymethylcellulose-Natrium, unlöslich quervernetzt | 3,0 mg |
| Magnesiumstearat | 1,2 mg |

### Herstellung:

Der Wirkstoff wird für 15 Minuten zusammen mit Lactose-monohydrat, mikrokristalliner Cellulose und Carboxymethylcellulose-Natrium in einem geeigneten Diffusionsmischer gemischt. Magnesiumstearat wird zugesetzt und für weitere 3 Minuten mit den übrigen Stoffen vermischt.

Die fertige Mischung wird auf einer Tablettenpresse zu runden, flachen Tabletten mit Facette verpreßt.
Durchmesser der Tablette: 7 mm
Gewicht einer Tablette: 120 mg

### Beispiel 8

### Kapseln mit 50 mg Wirkstoff pro Kapsel

### Zusammensetzung:

| | |
|---|---|
| Wirkstoff | 50,0 mg |
| Lactose-monohydrat | 130,0 mg |
| Maisstärke | 65,0 mg |
| Siliciumdioxid hochdispers | 2,5 mg |
| Magnesiumstearat | 2,5 mg |

### Herstellung:

Eine Stärkepaste wird hergestellt, indem ein Teil der Maisstärke mit einer geeigneten Menge heißen Wassers angequollen wird. Die Paste läßt man danach auf Zimmertemperatur abkühlen.

Der Wirkstoff wird in einem geeigneten Mischer mit Lactose-monohydrat und Maisstärke für 15 Minuten vorgemischt. Die Stärkepaste wird zugefügt und die Mischung wird ausreichend mit Wasser versetzt, um eine homogene feuchte Masse zu erhalten. Die feuchte Masse wird durch ein Sieb mit einer Maschenweite von 1,6 mm gegeben. Das gesiebte Granulat wird auf Horden bei etwa 55°C für 12 Stunden getrocknet.

Das getrocknete Granulat wird danach durch Siebe mit den Maschenweiten 1,2 und 0,8 mm gegeben. Hochdisperses Silicium wird in einem geeigneten Mischer in 3 Minuten mit dem Granulat vermischt. Danach wird Magnesiumstearat zugesetzt und für wietere 3 Minuten gemischt.

Die fertige Mischung wird mit Hilfe einer Kapselfüllmaschine in leere Kapselhüllen aus Hartgelatine der Größe 1 gefüllt.

### Beispiel 9

### Tabletten mit 200 mg Wirkstoff pro Tablette

### Zusammensetzung:

| | |
|---|---|
| Wirkstoff | 200,0 mg |
| Lactose-mMonohydrat | 167,0 mg |
| Microkristalline Cellulose | 80,0 mg |
| Hydroxypropyl-methylcellulose, Typ 2910 | 10,0 mg |
| Poly-1-vinyl-2-pyrrolidor, unlöslich quervernetzt | 20,0 mg |
| Magnesiumstearat | 3,0 mg |

### Herstellung:

HPMC wird in heißem Wasser dispergiert. Die Mischung ergibt nach dem Abkühlen eine klare Lösung.

Der Wirkstoff wird in einem geeigneten Mischer für 5 Minuten mit Lactose Monohydrat und mikrokristalliner Cellulose vorgemischt. Die HPMC- Lösung wird hinzugefügt und das Mischen fortgesetzt bis eine homogene feuchte Masse erhalten wird. Die feuchte Masse wird durch ein Sieb mit der Maschenweite 1,6 mm gegeben. Das gesiebte Granulat wird auf Horden bei etwa 55°C für 12 Stunden getrocknet.

Das getrocknete Granulat wird danach durch Siebe der Maschenweite 1,2 und 0,8 mm gegeben. Poly-1-vinyl-2-pyrrolidon wird in einem geeigneten Mischer für 3 Minuten mit dem Granulat vermischt. Danach wird Magnesiumstearat zugesetzt und für weitere 3 Minuten gemischt.

Die fertige Mischung wird auf einer Tablettenpresse zu oblongförmigen Tabletten verpreßt (16,2 x 7,9 mm) .
Gewicht einer Tablette: 480 mg

## Patentansprüche

1. Biphenylderivate der allgemeinen Formel 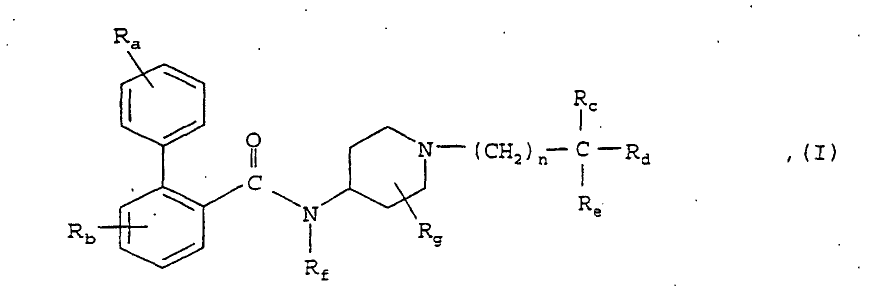 in der
n die Zahl 1, 2, 3, 4 oder 5,
Rₐ und R_{b}, die gleich oder verschieden sein können, jeweils ein Wasserstoff-, Fluor-, Chlor- oder Bromatom, eine C₁₋₃-Alkylgruppe, in der die Wasserstoffatome ganz oder teilweise durch Fluoratome ersetzt sein können, eine Hydroxy-, C₁₋₃-Alkoxy-, Amino- , C₁₋₃-Alkylamino- oder Di-(C₁₋₃-Alkyl)-aminogruppe,
R_{c} ein Wasserstoffatom,
eine C₁₋₁₀-Alkyl-, C₃₋₇-Cycloalkyl- oder C₃₋₇-Cycloalkyl-C₁₋₃-alkylgruppe, in denen jeweils die Wasserstoffatome ganz oder teilweise durch Fluoratome ersetzt sein können,
eine gegebenenfalls durch ein Fluor-, Chlor- oder Bromatom, durch eine C₁₋₃-Alkylgruppe, in der die Wasserstoffatome ganz oder teilweise durch Fluoratome ersetzt sein können, durch eine Hydroxy-, C₁₋₃-Alkoxy-, Carboxy-, C₁₋₃-Alkoxycarbonyl-, Aminocarbonyl-, C₁₋₃-Alkylaminocarbonyl- oder N,N-Di- (C₁₋₃-Alkyl)-aminocarbonylgruppe, durch eine 3- bis 7-gliedrige Cycloalkyleniminogruppe, wobei die Methylengruppe in Position 4 in einer 6- oder 7-gliedrigen Cycloalkyleniminogruppe zusätzlich durch ein Sauerstoff- oder Schwefelatom, durch eine Sulfinyl-, Sulfonyl-, Imino- oder N-(C₁₋₃-Alkyl)-iminogruppe ersetzt sein kann, durch eine Nitro-, Amino-, C₁₋₃-Alkylamino- Di- (C₁₋₃-Alkyl) -amino-, C₁₋₃-Alkylcarbonylamino-, N-(C₁₋₃-Alkyl)-C₁₋₃-alkylcarbonylamino-, C₁₋₃-Alkylsulfonylamino- oder N- (C₁₋₃-Alkyl)-C₁₋₃-alkylsulfonylaminogruppe substituierte Phenyl-, Naphthyloder monocyclische 5- oder 6-gliedrige Heteroarylgruppe, wobei die 6-gliedrige Heterdarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält, und außerdem an die vorstehend erwähnten monocyclischen heterocyclischen Gruppen über zwei benachbarte Kohlenstoffatome ein Phenylring ankondensiert sein kann,
R_{d} eine gegebenenfalls durch ein Fluor-, Chlor- oder Bromatom, durch eine C₁₋₃-Alkylgruppe, in der die Wasserstoffatome ganz oder teilweise durch Fluoratome ersetzt sein können, durch eine Hydroxy-, C₁₋₃-Alkoxy-, Carboxy-, C₁₋₃-Alkoxycarbonyl-, Aminocarbonyl-, C₁₋₃-Alkylaminocarbonyl- oder N,N-Di- (C₁₋₃-Alkyl)-aminocarbonylgruppe, durch eine 3- bis 7-gliedrige Cycloalkyleniminogruppe, wobei die Methylengruppe in Position 4 in einer 6- oder 7-gliedrigen Cycloalkyleniminogruppe zusätzlich durch ein Sauerstoff- oder Schwefelatom, durch eine Sulfinyl-, Sulfonyl-, Imino- oder N-(C₁₋₃-Alkyl)-iminogruppe ersetzt sein kann, durch eine Nitro-, Amino-, C₁₋₃-Alkylamino-, Di- (C₁₋₃-Alkyl) -amino-, C₁₋₃-Alkylcarbonylamino-, N-(C₁₋₃-Alkyl)-C₁₋₃-alkylcarbonylamino-, C₁₋₃-Alkylsulfonylamino- oder N- (C₁₋₃-Alkyl)-C₁₋₃-alkylsulfonylaminogruppe substituierte Phenyl-, Naphthyloder monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält, und außerdem an die vorstehend erwähnten monocyclischen heterocyclischen Gruppen über zwei benachbarte Kohlenstoffatome ein Phenylring ankondensiert sein kann,
Rₑ eine Carboxygruppe, eine C₁₋₆-Alkoxycarbonyl- oder C₃₋₇-Cycloalkoxycarbonylgruppe, in denen der Alkyl- oder Cycloalkylteil jeweils ab Position 2 bezogen auf das Sauerstoffatom durch eine C₁₋₃-Alkoxy-, Amino-, C₁₋₃-Alkylamino- oder Di-(C₁₋₃-alkyl)-aminogruppe substituiert sein kann, eine Phenyl-C₁₋₃-alkoxycarbonyl- oder Heteroaryl-C₁₋₃-alkoxycarbonylgruppe, wobei der Heteroarylteil wie vorstehend erwähnt definiert ist,
R_{f} ein Wasserstoffatom, eine C₁₋₃-Alkyl- oder Phenyl-C₁₋₃-alkylgruppe und
R_{g} ein Wasserstoffatom oder eine C₁₋₃-Alkylgruppe bedeuten,
deren Isomere und deren Salze.

2. Biphenylderivate der allgemeinen Formel I gemäß Anspruch 1, in der
R_{b} bis R_{g} wie im Anspruch 1 erwähnt definiert sind und
Rₐ in 3' oder 4'-Stellung steht und mit Ausnahme des Wasserstoffatoms wie eingangs erwähnt definiert ist,
deren Isomere und deren Salze.

3. Biphenylderivate der allgemeinen Formel I gemäß Anspruch 1, in der
n die Zahl 3, 4 oder 5,
Rₐ ein Wasserstoff-, Fluor-, Chlor- oder Bromatom, eine C₁₋₃-Alkyl-, Trifluormethyl- oder C₁₋₃-Alkoxygruppe,
R_{b} ein Wasserstoff-, Fluor-, Chlor- oder Bromatom,
R_{c} eine C₁₋₅-Alkyl-, C₃₋₇-Cycloalkyl- oder C₃₋₇-Cycloalkyl-C₂₋₃-alkylgruppe oder
eine gegebenenfalls durch ein Fluor-, Chlor- oder Bromatom oder durch eine C₁₋₃-Alkylgruppe substituierte Phenylgruppe,
R_{d} eine gegebenenfalls durch ein Fluor-, Chlor- oder Bromatom oder durch eine C₁₋₃-Alkylgruppe substituierte Phenylgruppe,
Rₑ eine Carboxygruppe, eine C₁₋₆-Alkoxycarbonyl- oder C₃₋₇-Cycloalkoxycarbonylgruppe, in denen der Alkyl- oder Cycloalkylteil jeweils ab Position 2 bezogen auf das Sauerstoffatom durch eine C₁₋₃-Alkoxy-, Amino-, C₁₋₃-Alkylamino- oder Di-(C₁₋₃-alkyl)-aminogruppe substituiert sein kann, eine Phenyl-C₁₋₃-alkoxycarbonyl-, Pyridyl-C₁₋₃-alkoxycarbonyl- oder Pyrimidyl-C₁₋₃-alkoxycarbonylgruppe,
R_{f} ein Wasserstoffatom oder eine C₁₋₃-Alkylgruppe und
R_{g} ein Wasserstoffatom oder. eine C₁₋₃-Alkylgruppe bedeuten,
deren Isomere und deren Salze.

4. Folgende Biphenylderivate der allgemeinen Formel I gemäß Anspruch 1:
(a) 2-Methyl-2-phenyl-5-{4-[(4'-trifluormethyl-biphenyl-2-carbonyl)-amino] -piperidin-1-yl)-pentancarbonsäure-methylester,
(b) 2-Ethyl-2-phenyl-5-{4-[(4'-trifluormethyl-biphenyl-2-carbonyl)-amino]-piperidin-1'-yl}-pentancarbonsäure-methylester und
(c) 2-Methyl-2-phenyl-6-{4-[(4'-trifluormethyl-biphenyl-2-carbonyl)-amino]-piperidin-1-yl}-hexancarbonsäuremethylester.

5. Physiologisch verträgliche Salze der Verbindungen gemäß den Ansprüchen 1 bis 3.

6. Arzneimittel, enthaltend eine Verbindung nach mindestens einem der Ansprüche 1 bis 4 oder ein Salz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 4 oder ein Salz gemäß Anspruch 5 zur Herstellung eines Arzneimittels mit einer senkenden Wirkung auf die Plasmaspiegel der atherogenen Lipoproteine.

8. Verfahren zur Herstellung eines Arzneimittels gemäß Anspruch 6, **dadurch gekennzeichnet, daß** auf nichtchemischem Wege eine Verbindung nach mindestens einem der Ansprüche 1 bis 4 oder ein Salz gemäß Anspruch 5 in einen oder mehrere inerte Trägerstoffe und/oder Verdünnungsmittel eingearbeitet wird.

9. Verfahren zur Herstellung der Verbindungen gemäß den Ansprüchen 1 bis 5, **dadurch gekennzeichnet, daß**
eine Verbindung der allgemeinen Formel 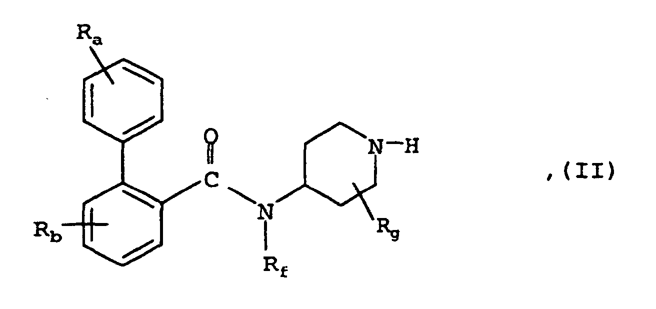 in der
Rₐ, R_{b}, R_{f} und R_{g} wie in den Ansprüchen 1 bis 4 erwähnt definiert sind, mit einer Verbindung der allgemeinen Formel 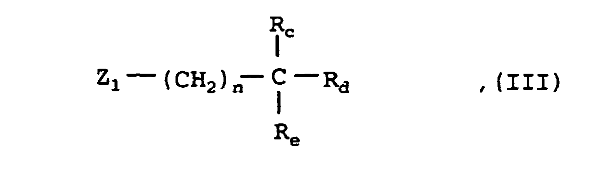 in der
n und R_{c} bis Rₑ wie in den Ansprüchen 1 bis 4 erwähnt definiert sind und
Z₁ eine nukleofuge Austrittsgruppe bedeutet, umgesetzt wird und
gewünschtenfalls anschließend eine so erhaltene Verbindung der allgemeinen Formel I, die eine Nitrogruppe enthält, mittels Reduktion in eine entsprechende Aminoverbindung übergeführt wird und/oder
eine so erhaltene Verbindung der allgemeinen Formel I, in der R_{f} ein Wasserstoffatom darstellt, mittels Alkylierung in eine entsprechende Verbindung, in der R_{f} eine C₁₋₃-Alkyl- oder Phenyl-C₁₋₃-alkylgruppe darstellt, übergeführt wird und/oder
ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

10. Verfahren zur Herstellung der Verbindungen gemäß den Ansprüchen 1 bis 5 mit der allgemeinen Formel I, in der Rₑ mit Ausnahme der Carboxygruppe die für Rₑ in den Ansprüchen 1 bis 4 erwähnten Bedeutungen aufweist, **dadurch gekennzeichnet, daß** eine Verbindung der allgemeinen Formel 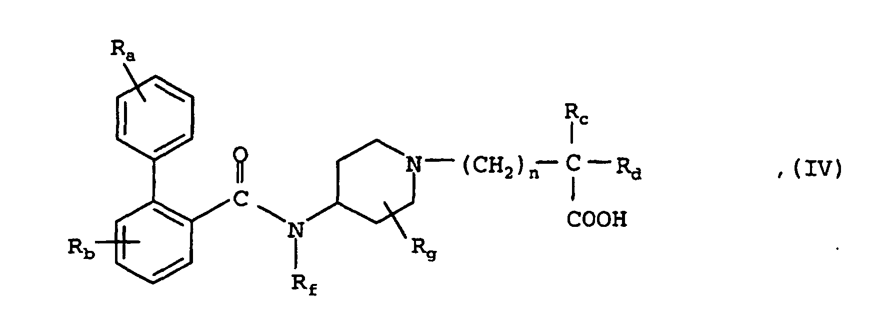 in der
n, Rₐ bis R_{d}, R_{f} und R_{g} wie in den Ansprüchen 1 bis 4 erwähnt definiert sind, oder deren reaktionsfähigen Derivate mit einem Alkohol der allgemeinen Formel
H - Rₑ' , (V)
in der
Rₑ' eine C₁₋₆-Alkoxy- oder C₃₋₇-Cycloalkoxygruppe, in denen der Alkyl- oder Cycloalkylteil jeweils ab Position 2 bezogen auf das Sauerstoffatom durch eine C₁₋₃-Alkoxy-, Amino-, C₁₋₃-Alkylamino- oder Di-(C₁₋₃-alkyl)-aminogruppe substituiert sein kann, eine Phenyl-C₁₋₃-alkoxy- oder Heteroaryl-C₁₋₃-alkoxygruppe, wobei der Heteroarylteil wie vorstehend erwähnt definiert ist, bedeutet, verestert wird oder zur Herstellung eines tert.Butylesters mit 2,2-Dimethyl-ethen in Gegegenwart einer Säure umgesetzt wird und
gewünschtenfalls anschließend eine so erhaltene Verbindung der allgemeinen Formel I, die eine Nitrogruppe enthält, mittels Reduktion in eine entsprechende Aminoverbindung übergeführt wird und/oder
eine so erhaltene Verbindung der allgemeinen Formel I, in der R_{f} ein Wasserstoffatom darstellt, mittels Alkylierung in eine entsprechende Verbindung, in der R_{f} eine C₁₋₃-Alkyl- oder Phenyl-C₁₋₃-alkylgruppe darstellt, übergeführt wird und/oder
ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

11. Verfahren zur Herstellung der Verbindungen gemäß den Ansprüchen 1 bis 5 mit allgemeinen Formel I, in der Rₑ eine Carboxygruppe darstellt, **dadurch gekennzeichnet, daß** eine Verbindung der allgemeinen Formel 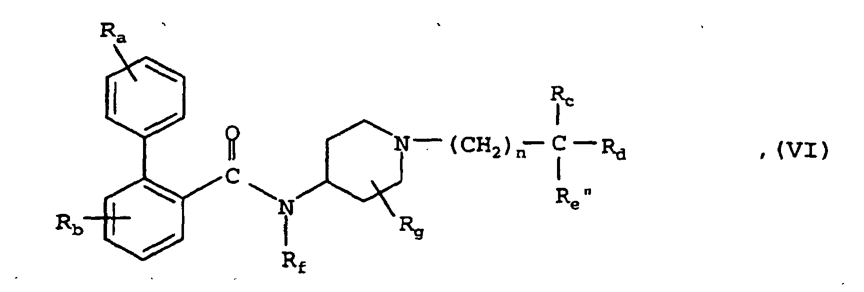 in der
n, Rₐ bis R_{d}, R_{f} und R_{g} wie in den Ansprüchen 1 bis 4 erwähnt definiert sind und
Rₑ" eine in eine Carboxygruppe überführbare Gruppe darstellt, in eine Verbindung der allgemeinen Formel I, in der Rₑ eine Carboxygruppe darstellt, übergeführt wird und gewünschtenfalls anschließend eine so erhaltene Verbindung der allgemeinen Formel I, die eine Nitrogruppe enthält, mittels Reduktion in eine entsprechende Aminoverbindung übergeführt wird und/oder
eine so erhaltene Verbindung der allagmeinen Formel I, in der R_{f} ein Wasserstoffatom darstellt, mittels Alkylierung in eine entsprechende Verbindung, in der R_{f} eine C₁₋₃-Alkyl- oder Phenyl-C₁₋₃-alkylgruppe darstellt, übergeführt wird und/oder
ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Biphenyl derivatives of general formula 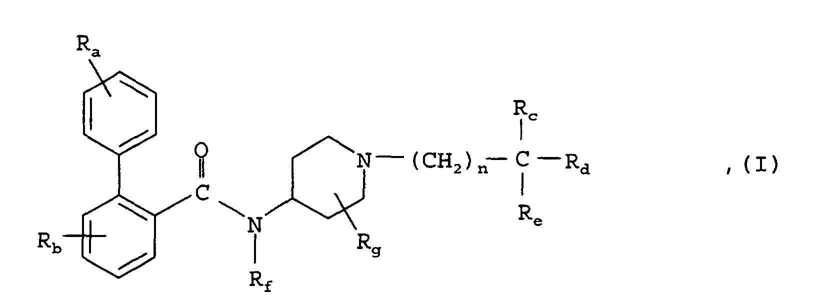 wherein
n denotes the number 1, 2, 3, 4 or 5,
Rₐ and R_{b}, which may be identical or different, each denote a hydrogen, fluorine, chlorine or bromine atom, a C₁₋₃-alkyl group wherein the hydrogen atoms may be wholly or partially replaced by fluorine atoms, a hydroxy, C₁₋₃-alkoxy, amino, C₁₋₃-alkylamino or di-(C₁₋₃-alkyl)-amino group,
R_{c} denotes a hydrogen atom,
a C₁₋₁₀-alkyl, C₃₋₇-cycloalkyl or C₃₋₇-cycloalkyl-C₁₋₃-alkyl group, wherein in each case the hydrogen atoms may be wholly or partially replaced by fluorine atoms,
a phenyl, naphthyl or monocyclic 5 or 6-membered heteroaryl group optionally substituted by a fluorine, chlorine or bromine atom, by a C₁₋₃-alkyl group wherein the hydrogen atoms may be wholly or partially replaced by fluorine atoms, by a hydroxy, C₁₋₃-alkoxy, carboxy, C₁₋₃-alkoxycarbonyl, aminocarbonyl, C₁₋₃-alkylaminocarbonyl or N,N-di-(C₁₋₃-alkyl)-aminocarbonyl group, by a 3- to 7-membered cycloalkyleneimino group, wherein the methylene group in the 4 position in a 6- or 7-membered cycloalkyleneimino group may additionally be replaced by an oxygen or sulphur atom, by a sulphinyl, sulphonyl, imino or N-(C₁₋₃-alkyl)-imino group, by a nitro, amino, C₁₋₃-alkylamino, di-(C₁₋₃-alkyl)-amino, C₁₋₃-alkylcarbonylamino, N-(C₁₋₃-alkyl)-C₁₋₃-alkylcarbonylamino, C₁₋₃-alkylsulphonylamino or N-(C₁₋₃-alkyl)-C₁₋₃-alkylsulphonylamino group, wherein the 6-membered heteroaryl group contains one, two or three nitrogen atoms and the 5-membered heteroaryl group contains an imino group optionally substituted by a C₁₋₃-alkyl group, an oxygen or sulphur atom, or an imino group optionally substituted by a C₁₋₃-alkyl group and an oxygen or sulphur atom or one or two nitrogen atoms, and moreover a phenyl ring may be fused to the abovementioned monocyclic heterocyclic groups via two adjacent carbon atoms,
R_{d} denotes a phenyl, naphthyl or monocyclic 5- or 6-membered heteroaryl group optionally substituted by a fluorine, chlorine or bromine atom, by a C₁₋₃-alkyl group wherein the hydrogen atoms may be wholly or partially replaced by fluorine atoms, by a hydroxy, C₁₋₃-alkoxy, carboxy, C₁₋₃-alkoxycarbonyl, aminocarbonyl, C₁₋₃-alkylaminocarbonyl or N,N-di-(C₁₋₃-alkyl)-aminocarbonyl group, by a 3- to 7-membered cycloalkyleneimino group, wherein the methylene group may additionally be replaced in the 4 position in a 6- or 7-membered cycloalkyleneimino group by an oxygen or sulphur atom, by a sulphinyl, sulphonyl, imino or N-(C₁₋₃-alkyl)-imino group, by a nitro, amino, C₁₋₃-alkylamino, di-(C₁₋₃-alkyl)-amino, C₁₋₃-alkylcarbonylamino, N-(C₁₋₃-alkyl)-C₁₋₃-alkylcarbonylamino, C₁₋₃-alkylsulphonylamino or N-(C₁₋₃-alkyl)-C₁₋₃-alkylsulphonylamino group, wherein the 6-membered heteroaryl group contains one, two or three nitrogen atoms and the 5-membered heteroaryl group contains an imino group optionally substituted by a C₁₋₃-alkyl group, an oxygen or sulphur atom, or an imino group optionally substituted by a C₁₋₃-alkyl group and an oxygen or sulphur atom or one or two nitrogen atoms, and moreover a phenyl ring may be fused to the abovementioned monocyclic heterocyclic groups via two adjacent carbon atoms,
Rₑ denotes a carboxy group, a C₁₋₆-alkoxycarbonyl or C₃₋₇-cycloalkoxycarbonyl group wherein the alkyl or cycloalkyl moiety may be substituted in each case from the 2 position, relative to the oxygen atom, by a C₁₋₃-alkoxy, amino, C₁₋₃-alkylamino or di-(C₁₋₃-alkyl)-amino group, a phenyl-C₁₋₃-alkoxycarbonyl or heteroaryl-C₁₋₃-alkoxycarbonyl group, while the heteroaryl moiety is as hereinbefore defined,
R_{f} denotes a hydrogen atom, a C₁₋₃-alkyl or phenyl-C₁₋₃-alkyl group and
R_{g} denotes a hydrogen atom or a C₁₋₃-alkyl group,
the isomers and salts thereof.

2. Biphenyl derivatives of general formula I according to claim 1, wherein
R_{b} to R_{g} are defined as in claim 1 and
Rₐ is in the 3' or 4' position and has the meanings given hereinbefore with the exception of the hydrogen atom,
the isomers and salts thereof.

3. Biphenyl derivatives of general formula I according to claim 1, wherein
n denotes the number 3, 4 or 5,
Rₐ denotes a hydrogen, fluorine, chlorine or bromine atom, a C₁₋₃-alkyl, trifluoromethyl or C₁₋₃-alkoxy group,
R_{b} denotes a hydrogen, fluorine, chlorine or bromine atom,
R_{c} denotes a C₁₋₅-alkyl, C₃₋₇-cycloalkyl or C₃₋₇-cycloalkyl-C₁₋₃-alkyl group or
a phenyl group optionally substituted by a fluorine, chlorine or bromine atom or by a C₁₋₃-alkyl group,
R_{d} denotes a phenyl group optionally substituted by a fluorine, chlorine or bromine atom or by a C₁₋₃-alkyl group,
Rₑ denotes a carboxy group, a C₁₋₆-alkoxycarbonyl or C₃₋₇-cycloalkoxycarbonyl group wherein the alkyl or cycloalkyl moiety may be substituted in each case from the 2 position, relative to the oxygen atom, by a C₁₋₃-alkoxy, amino, C₁₋₃-alkylamino or di-(C₁₋₃-alkyl)-amino group, a phenyl-C₁₋₃-alkoxycarbonyl, pyridyl-C₁₋₃-alkoxycarbonyl or pyrimidyl-C₁₋₃-alkoxycarbonyl group,
R_{f} denotes a hydrogen atom or a C₁₋₃-alkyl group and
R_{g} denotes a hydrogen atom or a C₁₋₃-alkyl group,
the isomers and salts thereof.

4. The following biphenyl derivatives of general formula I according to claim 1:
(a) methyl 2-methyl-2-phenyl-5-{4-[(4'-trifluoromethyl-biphenyl-2-carbonyl)-amino]-piperidin-1-yl}-pentanecarboxylate,
(b) methyl 2-ethyl-2-phenyl-5-{4-[(4'-trifluoromethyl-biphenyl-2-carbonyl)-amino]-piperidin-1-yl}-pentanecarboxylate and
(c) methyl 2-methyl-2-phenyl-6-{4-[(4'-trifluoromethyl-biphenyl-2-carbonyl)-amino]-piperidin-1-yl}-hexanecarboxylate.

5. Physiologically acceptable salts of the compounds according to claims 1 to 3.

6. Medicaments, containing a compound according to at least one of claims 1 to 4 or a salt according to claim 5 optionally together with one or more inert carriers and/or diluents.

7. Use of a compound according to at least one of claims 1 to 4 or a salt according to claim 5 for the preparation of a medicament having a lowering effect on the plasma levels of atherogenic lipoproteins.

8. Process for preparing a medicament according to claim 6, **characterised in that** a compound according to at least one of claims 1 to 4 or a salt according to claim 5 is incorporated in one or more inert carriers and/or diluents by a non-chemical method.

9. Process for preparing the compounds according to claims 1 to 5, **characterised in that**
a compound of general formula 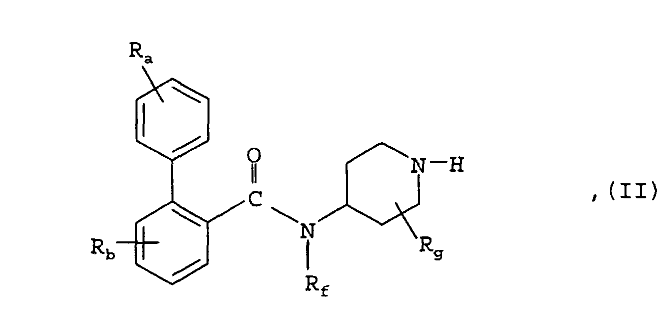 wherein
Rₐ, R_{b}, R_{f} and R_{g} are as defined in claims 1 to 4, is reacted with a compound of general formula 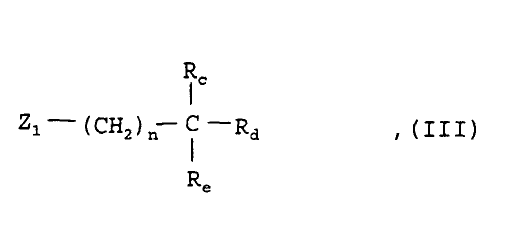 wherein
n and R_{c} to Rₑ are as defined in claims 1 to 4 and
Z₁ denotes a nucleofugic leaving group, and
subsequently, if desired, a compound of general formula I thus obtained which contains a nitro group is converted by reduction into a corresponding amino compound and/or
a compound of general formula I thus obtained wherein R_{f} denotes a hydrogen atom is converted by alkylation into a corresponding compound wherein R_{f} denotes a C₁₋₃-alkyl or phenyl-C₁₋₃-alkyl group, and/or
a protecting group used during the reactions to protect reactive groups is cleaved and/or
a compound of general formula I thus obtained is resolved into its stereoisomers and/or
a compound of general formula I thus obtained is converted into the salts thereof, particularly for pharmaceutical use into the physiologically acceptable salts with an inorganic or organic acid or base.

10. Process for preparing the compounds according to claims 1 to 5 having the general formula I, wherein Rₑ has the meanings mentioned for Rₑ in claims 1 to 4 with the exception of the carboxy group, **characterised in that** a compound of general formula 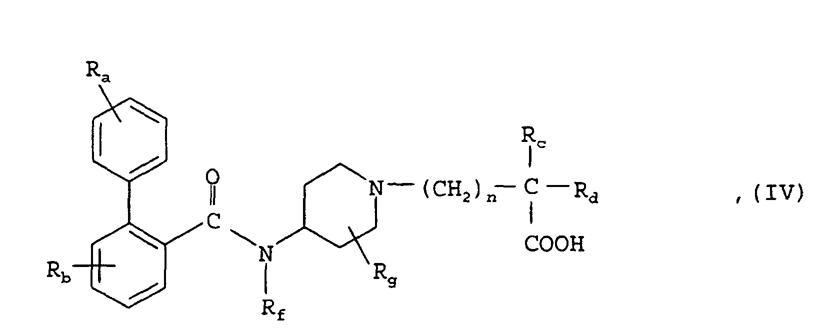 wherein
n, Rₐ to R_{d}, R_{f} and R_{g} are as defined in claims 1 to 4, or the reactive derivatives thereof, is esterified with an alcohol of general formula
H - Rₑ' , (V)
wherein
Rₑ' denotes a C₁₋₆-alkoxy or C₃₋₇-cycloalkoxy group wherein the alkyl or cycloalkyl moiety may in each case be substituted from the 2 position, relative to the oxygen atom, by a C₁₋₃-alkoxy, amino, C₁₋₃-alkylamino or di-(C₁₋₃-alkyl)-amino group, or Rₑ' denotes a phenyl-C₁₋₃-alkoxy or heteroaryl-C₁₋₃-alkoxy group, while the heteroaryl moiety is as hereinbefore defined, or
in order to prepare a tert.butylester it is reacted with 2,2-dimethyl-ethene in the presence of an acid, and
subsequently, if desired, a compound of general formula I thus obtained which contains a nitro group is converted by reduction into a corresponding amino compound and/or
a compound of general formula I thus obtained wherein R_{f} denotes a hydrogen atom is converted by alkylation into a corresponding compound wherein R_{f} denotes a C₁₋₃-alkyl or phenyl-C₁₋₃-alkyl group, and/or
a protecting group used during the reactions to protect reactive groups is cleaved and/or
a compound of general formula I thus obtained is resolved into its stereoisomers and/or
a compound of general formula I thus obtained is converted into the salts thereof, particularly for pharmaceutical use into the physiologically acceptable salts with an inorganic or organic acid or base.

11. Process for preparing the compounds according to claims 1 to 5 having the general formula I, wherein Rₑ denotes a carboxy group, **characterised in that** a compound of general formula 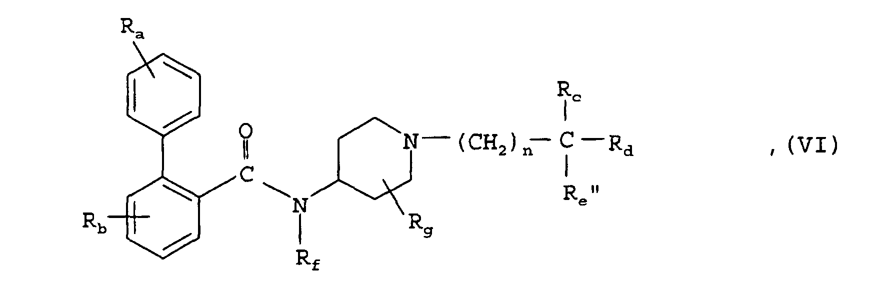 wherein
n, Rₐ to R_{d}, R_{f} and R_{g} are as defined in claims 1 to 4 and
Rₑ" denotes a group which can be converted into a carboxy group, is converted into a compound of general formula I wherein Rₑ denotes a carboxy group, and
subsequently, if desired, a compound of general formula I thus obtained which contains a nitro group is converted by reduction into a corresponding amino compound and/or
a compound of general formula I thus obtained wherein R_{f} denotes a hydrogen atom is converted by alkylation into a corresponding compound wherein R_{f} denotes a C₁₋₃-alkyl or phenyl-C₁₋₃-alkyl group, and/or
a protecting group used during the reactions to protect reactive groups is cleaved and/or
a compound of general formula I thus obtained is resolved into its stereoisomers and/or
a compound of general formula I thus obtained is converted into the salts thereof, particularly for pharmaceutical use into the physiologically acceptable salts with an inorganic or organic acid or base.

## Revendications

1. Dérivés de biphényle de formule générale 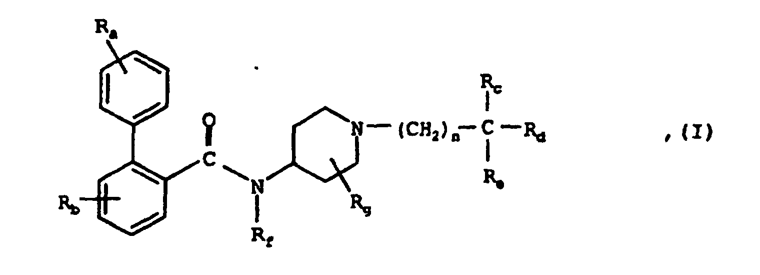 où
n représente le nombre 1, 2, 3, 4 ou 5,
Rₐ et R_{b}, qui peuvent être identiques ou différents, représentent chacun un atome d'hydrogène, de fluor, de chlore ou de brome, un groupe C₁₋₃-alkyle dans lequel les atomes d'hydrogène peuvent être remplacés en totalité ou en partie par des atomes de fluor, un groupe hydroxyle, C₁₋₃-alcoxy, amino, C₁₋₃-alkylamino ou di-(C₁₋₃-alkyl)-amino,
R_{c} représente un atome d'hydrogène,
un groupe C₁₋₁₀-alkyle, C₃₋₇-cycloalkyle ou C₃₋₇-cycloalkyle-C₁₋₃-alkyle, dans lesquels à chaque fois les atomes d'hydrogène peuvent être remplacés en totalité ou en partie par des atomes de fluor,
un groupe phényle, naphtyle ou hétéroaryle à 5 ou 6 chaînons monocyclique éventuellement substitué par un atome de fluor, de chlore ou de brome, par un groupe C₁₋₃-alkyle, dans lequel les atomes d'hydrogène peuvent être remplacés en totalité ou en partie par des atomes de fluor, par un groupe hydroxyle, C₁₋₃-alcoxy, carboxyle, C₁₋₃-alcoxycarbonyle, aminocarbonyle, C₁₋₃-alkylaminocarbonyle ou N,N-di-(C₁₋₃-alkyl)-aminocarbonyle, par un groupe cycloalkylèneimino à 3 à 7 chaînons, où le groupe méthylène en position 4 d'un groupe cycloalkylèneimino à 6 ou 7 chaînons peuvent être remplacé en outre par un atome d'oxygène ou de soufre, par un groupe sulfinyle, sulfonyle, imino ou N-(C₁₋₃-alkyle)-imino, par un groupe nitro, amino, C₁₋₃-alkylamino, di-(C₁₋₃-alkyl)-amino, C₁₋₃-alkylcarbonylamino, N-(C₁₋₃-alkyl)-C₁₋₃-alkylcarbonylamino, C₁₋₃-alkylsulfonylamino ou N-(C₁₋₃-alkyl)-C₁₋₃-alkylsulfonylamino, où le groupe hétéroaryle à 6 chaînons contient un, deux ou trois atomes d'azote et le groupe hétéroaryle à 5 chaînons contient un groupe imino éventuellement substitué par un groupe C₁₋₃-alkyle, un atome d'oxygène ou un atome de soufre ou un groupe imino éventuellement substitué par un groupe C₁₋₃-alkyle et un atome d'oxygène ou un atome de soufre ou un ou deux atomes d'azote, et en outre un cycle phényle peut être condensé aux groupes hétérocycliques monocycliques mentionnés précédemment par deux atomes de carbone voisins,
R_{d} représente un groupe phényle, naphtyle ou hétéroaryle à 5 ou 6 chaînons monocyclique éventuellement substitué par un atome de fluor, de chlore ou de brome, par un groupe C₁₋₃-alkyle, dans lequel les atomes d'hydrogène peuvent être remplacés en totalité ou en partie par des atomes de fluor, par un groupe hydroxyle, C₁₋₃-alcoxy, carboxyle, C₁₋₃-alcoxycarbonyle, aminocarbonyle, C₁₋₃-alkylaminocarbonyle ou N,N-di-(C₁₋₃-alkyl)-aminocarbonyle, par un groupe cycloalkylèneimino à 3 à 7 chaînons, où le groupe méthylène en position 4 d'un groupe cycloalkylèneimino à 6 ou 7 chaînons peuvent être remplacé en outre par un atome d'oxygène ou de soufre, par un groupe sulfinyle, sulfonyle, imino ou N-(C₁₋₃-alkyle)-imino, par un groupe nitro, amino, C₁₋₃-alkylamino, di-(C₁₋₃-alkyl)-amino, C₁₋₃-alkylcarbonylamino, N-(C₁₋₃-alkyl)-C₁₋₃-alkylcarbonylamino, C₁₋₃-alkylsulfonylamino ou N-(C₁₋₃-alkyl)-C₁₋₃-alkylsulfonylamino, où le groupe hétéroaryle à 6 chaînons contient un, deux ou trois atomes d'azote et le groupe hétéroaryle à 5 chaînons contient un groupe imino éventuellement substitué par un groupe C₁₋₃-alkyle, un atome d'oxygène ou un atome de soufre ou un groupe imino éventuellement substitué par un groupe C₁₋₃-alkyle et un atome d'oxygène ou un atome de soufre ou un ou deux atomes d'azote, et en outre un cycle phényle peut être condensé aux groupes hétérocycliques monocycliques mentionnés précédemment par deux atomes de carbone voisins,
Rₑ représente un groupe carboxyle, un groupe C₁₋₆-alcoxycarbonyle ou C₃₋₇-cycloalcoxycarbonyle, dans lesquels la partie alkyle ou cycloalkyle peut être substituée à chaque fois à partir de la position 2 par rapport à l'atome d'oxygène par un groupe C₁₋₃-alcoxy, amino, C₁₋₃-alkylamino ou di-(C₁₋₃-alkyl)-amino, un groupe phényl-C₁₋₃-alcoxycarbonyle ou hétéroaryl-C₁₋₃-alcoxycarbonyle, où la partie hétéroaryle est définie comme indiqué précédemment,
R_{f} représente un atome d'hydrogène, un groupe C₁₋₃-alkyle ou phényl- C₁₋₃-alkyle et
R_{g} représente un atome d'hydrogène ou un groupe C₁₋₃-alkyle,
leurs isomères et leurs sels.

2. Dérivés de biphényle de formule générale I selon la revendication 1 où
R_{b} à R_{g} sont définis comme dans la revendication 1 et
Rₐ est situé en position 3' ou 4' et est défini comme indiqué au début à l'exception de l'atome d'hydrogène,
leurs isomères et leurs sels.

3. Dérivés de biphényle de formule générale I selon la revendication 1 où
n représente le nombre 3, 4 ou 5,
Rₐ représente un atome d'hydrogène, de fluor, de chlore ou de brome, un groupe C₁₋₃-alkyle, trifluorométhyle ou C₁₋₃-alcoxy,
R_{b} représente un atome d'hydrogène, de fluor, de chlore ou de brome,
R_{c} représente un groupe C₁₋₅-alkyle, C₃₋₇-cycloalkyle ou C₃₋₇-cycloalkyl-C₁₋₃-alkyle ou
un groupe phényle éventuellement substitué par un atome de fluor, de chlore ou de brome ou par un groupe C₁₋₃-alkyle,
R_{d} représente un groupe phényle éventuellement substitué par un atome de fluor, de chlore ou de brome ou par un groupe C₁₋₃-alkyle,
Rₑ représente un groupe carboxyle, un groupe C₁₋₆-alcoxycarbonyle ou C₃₋₇-cycloalcoxycarbonyle, dans lesquels la partie alkyle ou cycloalkyle peut être substituée à chaque fois à partir de la position 2 par rapport à l'atome d'oxygène par un groupe C₁₋₃-alcoxy, amino, C₁₋₃-alkylamino ou di-(C₁₋₃-alkyl)-amino, un groupe phényl-C₁₋₃-alcoxycarbonyle, pyridyl-C₁₋₃-alcoxycarbonyle ou pyrimidyl-C₁₋₃-alcoxycarbonyle,
R_{f} représente un atome d'hydrogène ou un groupe C₁₋₃-alkyle et
R_{g} représente un atome d'hydrogène ou un groupe C₁₋₃-alkyle,
leurs isomères et leurs sels.

4. Dérivés de biphényle de formule générale I selon la revendication 1 suivants :
(a) 2-méthyl-2-phényl-5-{4-[(4'-trifluorométhyl-biphényl-2-carbonyl)-amino]-pipéridin-1-yl}-pentanecarboxylate de méthyle,
(b) 2-éthyl-2-phényl-5-{4-[(4'-trifluorométhyl-biphényl-2-carbonyl)-amino]-pipéridin-1-yl}-pentanecarboxylate de méthyle,
et
(c) 2-méthyl-2-phényl-6-{4-[(4'-trifluorométhyl-biphényl-2-carbonyl)-amino]-pipéridin-1-yl}-hexanecarboxylate de méthyle.

5. Sels physiologiquement acceptables des composés selon les revendications 1 à 3.

6. Médicament contenant un composé selon au moins l'une des revendications 1 à 4 ou un sel selon la revendication 5 outre éventuellement un ou plusieurs supports et/ou diluants inertes.

7. Utilisation d'un composé selon au moins l'une des revendications 1 à 4 ou d'un sel selon la revendication 5 pour la production d'un médicament ayant un effet réducteur sur la concentration plasmatique des lipoprotéines athérogènes.

8. Procédé de production d'un médicament selon la revendication 6 **caractérisé en ce que**, par voie non chimique, un composé selon au moins l'une des revendications 1 à 4 ou un sel selon la revendication 5 est incorporé dans un ou plusieurs supports et/ou diluants inertes.

9. Procédé de production des composés selon les revendications 1 à 5 **caractérisé en ce que**
un composé de formule générale 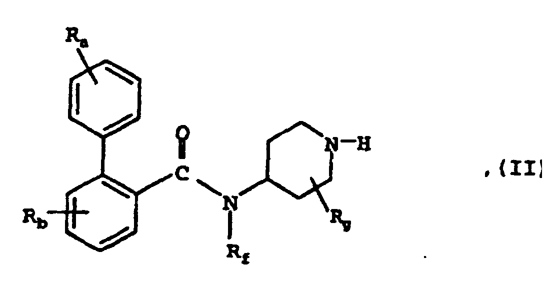 où
Rₐ, R_{b}, R_{f} et R_{g} sont définis comme indiqué dans les revendications 1 à 4, est mis à réagir avec un composé de formule générale 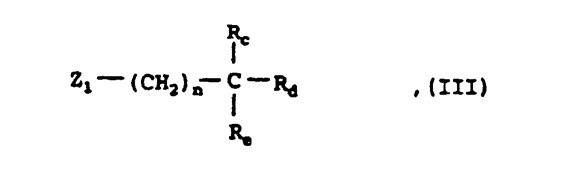 où
n et R_{c} à Rₑ sont définis comme indiqué dans les revendications 1 à 4 et
Z₁ représente un groupe partant nucléofuge, puis,
si on le souhaite, un composé de formule générale I ainsi obtenu, qui contient un groupe nitro, est éventuellement converti par réduction en un composé amino correspondant et/ou
un composé de formule générale I ainsi obtenu, où R_{f} représente un atome d'hydrogène, est converti par alkylation en un composé correspondant où R_{f} représente un groupe C₁₋₃-alkyle ou phényl-C₁₋₃-alkyle, et/ou
un groupement protecteur utilisé pendant les réactions pour la protection de groupes réactifs est clivé et/ou
un composé de formule générale I ainsi obtenu est séparé en ses stéréoisomères et/ou
un composé de formule générale I ainsi obtenu est converti en ses sels, en particulier pour l'utilisation pharmaceutique, en ses sels physiologiquement acceptables avec un acide ou base inorganique ou organique.

10. Procédé de production des composés selon les revendications 1 à 5 ayant la formule générale 1 où Rₑ, à l'exception du groupe carboxyle, présente les significations indiquées pour Rₑ dans les revendications 1 à 4, **caractérisé en ce qu'**un composé de formule générale 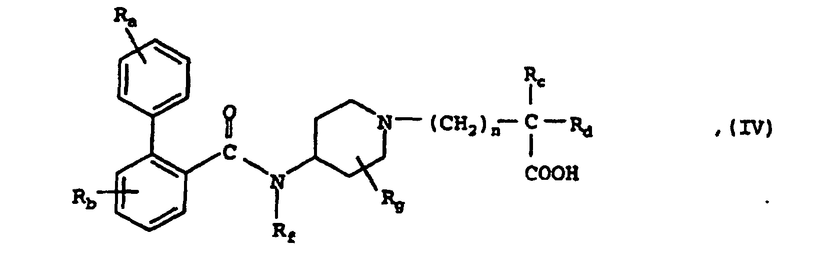 où
n, Rₐ à R_{d}, R_{f} et R_{g} sont définis comme indiqué dans les revendications 1 à 4, ou ses dérivés réactifs, est estérifié avec un alcool de formule générale
H - Rₑ' (V)
où Rₑ' représente un groupe C₁₋₆-alcoxy ou C₃₋₇-cycloalcoxy, dans lesquels la partie alkyle ou cycloalkyle peut être substituée à chaque fois à partir de la position 2 par rapport à l'atome d'oxygène par un groupe C₁₋₃-alcoxy, amino, C₁₋₃-alkylamino ou di-(C₁₋₃-alkyl)-amino, un groupe phényl-C₁₋₃-alcoxy ou hétéroaryl-C₁₋₃-alcoxy, où la partie hétéroaryle est définie comme indiqué précédemment, ou pour la préparation d'un tert.butylester est mis à réagir avec le 2,2-diméthyl-éthène en présence d'un acide puis,
si on le souhaite, un composé de formule générale I ainsi obtenu, qui contient un groupe nitro, est éventuellement converti par réduction en un composé amino correspondant et/ou
un composé de formule générale 1 ainsi obtenu, où R_{f} représente un atome d'hydrogène, est converti par alkylation en un composé correspondant où R_{f} représente un groupe C₁₋₃-alkyle ou phényl-C₁₋₃-alkyle, et/ou
un groupement protecteur utilisé pendant les réactions pour la protection de groupes réactifs est clivé et/ou
un composé de formule générale I ainsi obtenu est séparé en ses stéréoisomères et/ou
un composé de formule générale I ainsi obtenu est converti en ses sels, en particulier pour l'utilisation pharmaceutique, en ses sels physiologiquement acceptables avec un acide ou base inorganique ou organique.

11. Procédé de production des composés selon les revendications 1 à 5 ayant la formule générale 1 où Rₑ représente un groupe carboxyle, **caractérisé en ce qu'**un composé de formule générale 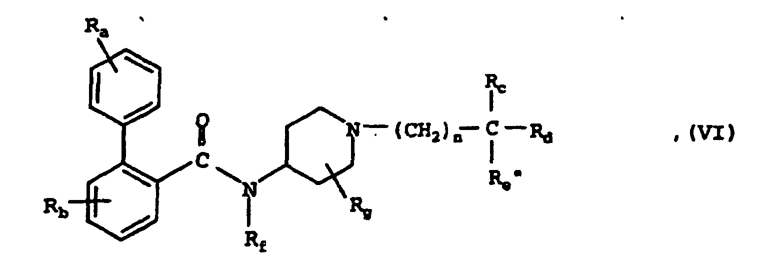 où
n, Rₐ à R_{d}, R_{f} et R_{g} sont définis comme indiqué dans les revendications 1 à 4 et
Rₑ" représente un groupe qui peut être converti en un groupe carboxyle, est converti en un composé de formule générale I où Rₑ représente un groupe carboxyle puis,
si on le souhaite, un composé de formule générale I ainsi obtenu, qui contient un groupe nitro, est éventuellement converti par réduction en un composé amino correspondant et/ou
un composé de formule générale I ainsi obtenu, où R_{f} représente un atome d'hydrogène, est converti par alkylation en un composé correspondant où R_{f} représente un groupe C₁₋₃-alkyle ou phényl-C₁₋₃-alkyle, et/ou
un groupement protecteur utilisé pendant les réactions pour la protection de groupes réactifs est clivé et/ou
un composé de formule générale I ainsi obtenu est séparé en ses stéréoisomères et/ou
un composé de formule générale I ainsi obtenu est converti en ses sels, en particulier pour l'utilisation pharmaceutique, en ses sels physiologiquement acceptables avec un acide ou base inorganique ou organique.
